# EUROPEAN PATENT APPLICATION

(11) **EP 1 602 666 A1**
(43) Date of publication of application: **07.12.2005**
(21) Application number: 04718763.8
(22) Date of filing: 09.03.2004
(51) Int. Cl.: C07K 14/47, C12N 15/12, C12N 5/10, C12N 1/15, C12N 1/19, C12N 1/21, C12Q 1/02, C07K 16/18, C12P 21/02, A61K 31/7088, A61K 45/00, A61K 48/00, A61P 3/10, A61P 21/02, A61P 25/14, A61P 25/16, A61P 25/28, G01N 33/15, G01N 33/50, G01N 33/53, G01N 33/566

(54) **NOVEL PROTEIN AND ITS DNA**

(30) Priority: 10.03.2003 JP 2003064197; 27.05.2003 JP 2003149679
(71) Applicant: Takeda Chemical Industries, Ltd., Osaka-shi, Osaka 541-0045 (JP)
(72) Inventor: HORIGUCHI, Takashi, c/o Takeda Pharm. Co. Ltd, Ibaraki, 300-4293 (JP); MATSUI, Hideki, c/o Takeda Pharm. Co. Ltd, Osaka-shi,Osaka 532-8686 (JP); WATANABE, Tomomichi, c/o Takeda Pharm. Co. Ltd, Ibaraki, 300-4293 (JP)
(74) Representative: Lewin, John Harvey
(86) International application number: PCT/JP2004/003033
(87) International publication number: WO 2004/081039

(57) **Abstract**

A protein, a polynucleotide, an antibody of the present invention, etc. are useful as, for example, a diagnostic marker for a neurodegenerative disease and so on. A compound promoting or inhibiting the activity of the protein, a compound promoting or inhibiting the expression of a gene for the above protein, etc., which are obtained by a screening method with the use of the protein, polynucleotide or antibody as described above, are usable as, for example, a prophylactic/therapeutic agent for a neurodegenerative disease and so on.

## Description

### TECHNICAL FIELD

The present invention provides a novel protein, a polynucleotide encoding the protein, a method of screening a compound promoting or inhibiting the activity of the protein, a compound obtained by the screening method, etc. More specifically, the present invention relates to a prophylactic/therapeutic agent for a neurodegenerative disease, a β-amyloid production inhibitor, an cell-death inhibitor and a diagnostic agent.

### BACKGROUND ART

Alzheimer's disease is a typical neurodegenerative disease accompanied by progressive dementia and loss in cognitive ability, but an effective therapeutic method therefor has never been found. It goes without saying that Alzheimer's disease is the most serious disease at the present time gradually reaching an aging society, and the development of a therapeutic agent therefor also has an extremely significant meaning from medical and economical points of view.

On one hand, it is known that by factors such as heat shock and glucose starvation influencing the biosynthesis of proteins, abnormal proteins are accumulated in endoplasmic reticula, thus stressing the endoplasmic reticula (endoplasmic reticulum stress). When the living body is subjected to endoplasmic reticulum stress, endoplasmic reticulum response genes including a chaperon molecule are expressed to repair or decompose the abnormal proteins thereby maintaining homeostasis.

In recent years, the relationship between various neurodegenerative diseases and endoplasmic reticulum stress has been regarded important. Parkin was identified as a causative gene of autosomal recessive inherited juvenile parkinsonism (AR-JP) that is inherited Parkinson's disease (Nature, 392, 605-608, 1998), and reported to be ubiquitin ligase involved in proteolysis system (Nat. Genet, 25, 302-305, 2000). As a substrate for Parkin, a Pael (Parkin associated endothelin receptor-like) receptor was identified. This receptor is a protein hardly forming a higher-order structure, and this protein of incomplete higher-order structure is usually rapidly decomposed by the action of Parkin, but when the proteolysis system is suppressed, abnormal Pael receptor is accumulated in endoplasmic reticula, and the cell falls into cell-death due to endoplasmic reticulum stress (Cell, 105, 891-902, 2001). It is also reported that a cell having an abnormality in preselinin 1 as a causative gene of familial Alzheimer's disease is made vulnerable to endoplasmic reticulum stress, and production of β-amyloid is increased due to lack of Ire1 participating in endoplasmic reticulum stress response (Biochem. Biophysic. Acta. 1536, 85-96, 2001; J. Biol. Chem., 276, 2108-2114, 2001).

There is demand for a safe and excellent prophylactic/therapeutic agent for neurodegenerative diseases.

### DISCLOSURE OF INVENTION

To solve the problem described above, the present inventors estimated that a target gene for creating a medicine for neurodegenerative diseases can be found in genes participating in endoplasmic reticulum stress response, and they made an exhaustive analysis of gene expression in nerve cells against endoplasmic reticulum stress, and as a result of extensive study, they found a novel gene whose expression was significantly increased upon application of endoplasmic reticulum stress to nerve cells. A protein of this gene agreed in the C-terminal region with a hypothetical protein, Homo sapiens, hypothetical protein MGC4504 (Genbank No. BC019625). On the basis of this finding, the present inventors made further examination to complete the present invention.

That is, the present invention relates to:
(1) a protein comprising the same or substantially the same amino acid sequence as an amino acid sequence represented by SEQ ID NO: 1, or a salt thereof,
(2) a protein consisting of an amino acid sequence represented by SEQ ID NO: 1, or a salt thereof,
(3) a partial peptide of the protein according to the above-mentioned (1), or a salt thereof.
(4) a polynucleotide comprising a polynucleotide encoding the protein according to the above-mentioned (1) or the partial peptide according to the above-mentioned (3),
(5) the polynucleotide according to the above-mentioned (4), which is DNA,
(6) a polynucleotide consisting of a nucleotide sequence represented by SEQ ID NO: 2,
(7) a recombinant vector comprising the polynucleotide according to the above-mentioned (5),
(8) a transformant transformed with the recombinant vector according to the above-mentioned (7),
(9) a method of manufacturing the protein or its salt according to the above-mentioned (1) or the partial peptide or its salt according to the above-mentioned (3), which comprises culturing the transformant according to the above-mentioned (8), forming and accumulating the protein according to the above-mentioned (1) or the partial peptide according to the above-mentioned (3), and recovering it,
(10) a pharmaceutical comprising the protein or its salt according to the above-mentioned (1) or the partial peptide or its salt according to the above-mentioned (3),
(10a) the pharmaceutical according to the above-mentioned (10), which is a prophylactic/therapeutic agent for a neurodegenerative disease,
(10b) the pharmaceutical according to the above-mentioned (10), which is a β-amyloid production inhibitor,
(11) a pharmaceutical comprising the polynucleotide according to the above-mentioned (5),
(11a) the pharmaceutical according to the above-mentioned (11), which is a prophylactic/therapeutic agent for a neurodegenerative disease,
(11b) the pharmaceutical according to the above-mentioned (11), which is a β-amyloid production inhibitor,
(12) a diagnostic agent comprising the polynucleotide according to the above-mentioned (5),
(12a) the diagnostic agent according to the above-mentioned (12), which is a diagnostic agent for a neurodegenerative disease,
(13) an antibody to the protein according to the above-mentioned (1), the partial peptide according to the above-mentioned (3), or a salt of the protein or partial peptide,
(14) an antisense polynucleotide comprising a nucleotide sequence, or a part thereof, complementary or substantially complementary to the nucleotide sequence of a polynucleotide encoding a protein or its partial peptide thereof comprising the same or substantially the same amino acid sequence as an amino acid sequence represented by SEQ ID NO: 1,
(15) a pharmaceutical comprising the antisense polynucleotide according to the above-mentioned (14),
(15a) the pharmaceutical according to the above-mentioned (15), which is an cell-death inhibitor,
(16) a method of screening a compound or its salt that promotes or inhibits the activity of the protein or its salt according to the above-mentioned (1) or the partial peptide or its salt according to the above-mentioned (3), which comprises using the protein or its salt according to the above-mentioned (1) or the partial peptide or its salt according to the above-mentioned (3),
(17) a kit for screening a compound or its salt that promotes or inhibits the activity of the protein or its salt according to the above-mentioned (1) or the partial peptide or its salt according to the above-mentioned (3), which comprises the protein or its salt according to the above-mentioned (1) or the partial peptide or its salt according to the above-mentioned (3),
(17a) a compound or its salt that promotes the activity of the protein or its salt according to the above-mentioned (1) or the partial peptide or its salt according to the above-mentioned (3), which is obtained by using the screening method according to the above-mentioned (16) or the screening kit according to the above-mentioned (17),
(17b) a compound or its salt that inhibits the activity of the protein or its salt according to the above-mentioned (1) or the partial peptide or its salt according to the above-mentioned (3), which is obtained by using the screening method according to the above-mentioned (16) or the screening kit according to the above-mentioned (17),
(17c) a pharmaceutical comprising the compound or its salt according to the above-mentioned (17a),
(17d) a pharmaceutical comprising the compound or its salt according to the above-mentioned (17b),
(17e) the pharmaceutical according to the above-mentioned (17c), which is a β-amyloid production inhibitor,
(17f) the pharmaceutical according to the above-mentioned (17d), which is an cell-death inhibitor,
(17g) the pharmaceutical according to the above-mentioned (17c), which is a prophylactic/therapeutic agent for a neurodegenerative disease,
(17h) the pharmaceutical according to the above-mentioned (17g), wherein the neurodegenerative disease is Alzheimer's disease (familial Alzheimer's disease, juvenile Alzheimer's disease, sporadic Alzheimer's disease, mild cognitive impairment etc.), cerebral amyloid angiopathy, Parkinson's disease or Down's syndrome,
(17i) the pharmaceutical according to the above-mentioned (17d), which is a prophylactic/therapeutic agent for a neurodegenerative disease,
(18) a method of screening a compound or its salt that promotes or inhibits the expression of a gene for the protein according to the above-mentioned (1), which comprises using the polynucleotide according to the above-mentioned (4),
(19) a kit for screening a compound or its salt that promotes or inhibits the expression of a gene for the protein according to the above-mentioned (1), which comprises the polynucleotide according to the above-mentioned (4),
(19a) a compound or its salt that promotes the expression of a gene for the protein according to the above-mentioned (1), which is obtained by using the screening method according to the above-mentioned (18) or the screening kit according to the above-mentioned (19),
(19b) a compound or its salt that inhibits the expression of a gene for the protein according to the above-mentioned (1), which is obtained by using the screening method according to the above-mentioned (18) or the screening kit according to the above-mentioned (19),
(19c) a pharmaceutical comprising the compound or its salt according to the above-mentioned (19a),
(19d) a pharmaceutical comprising the compound or its salt according to the above-mentioned (19b),
(19e) the pharmaceutical according to the above-mentioned (19c), which is a β-amyloid production inhibitor,
(19f) the pharmaceutical according to the above-mentioned (19d), which is an cell-death inhibitor,
(19g) the pharmaceutical according to the above-mentioned (19c), which is a prophylactic/therapeutic agent for a neurodegenerative disease,
(19h) the pharmaceutical according to the above-mentioned (19g), wherein the neurodegenerative disease is Alzheimer's disease (familial Alzheimer's disease, juvenile Alzheimer's disease, sporadic Alzheimer's disease, mild cognitive impairment etc.), cerebral amyloid angiopathy, Parkinson's disease or Down's syndrome,
(19i) the pharmaceutical according to the above-mentioned (19d), which is a prophylactic/therapeutic agent for a neurodegenerative disease,
(20) a diagnostic agent comprising the antibody according to the above-mentioned (13),
(21) a pharmaceutical comprising the antibody according to the above-mentioned (13),
(22) a method of quantifying the protein according to the above-mentioned (1), which comprises using the antibody according to the above-mentioned (13),
(23) a method of diagnosing diseases associated with the functions of the protein according to the above-mentioned (1), which comprises using the quantification method according to the above-mentioned (22),
(24) a method of screening a compound or its salt that promotes or inhibits the expression of the protein according to the above-mentioned (1), which comprises using the antibody according to the above-mentioned (13),
(25) a kit for screening a compound or its salt that promotes or inhibits the expression of the protein according to the above-mentioned (1), which comprises the antibody according to the above-mentioned (13),
(25a) a compound or its salt that promotes the expression of the protein according to the above-mentioned (1), which is obtained by using the screening method according to the above-mentioned (24) or the screening kit according to the above-mentioned (25),
(25b) a compound or its salt that inhibits the expression of the protein according to the above-mentioned (1), which is obtained by using the screening method according to the above-mentioned (24) or the screening kit according to the above-mentioned (25),
(25c) a pharmaceutical comprising the compound or its salt according to the above-mentioned (25a),
(25d) a pharmaceutical comprising the compound or its salt according to the above-mentioned (25b),
(25e) the pharmaceutical according to the above-mentioned (25c), which is a β-amyloid production inhibitor,
(25f) the pharmaceutical according to the above-mentioned (25d), which is an cell-death inhibitor,
(25g) the pharmaceutical according to the above-mentioned (25c), which is a prophylactic/therapeutic agent for a neurodegenerative disease,
(25h) the pharmaceutical according to the above-mentioned (25g), wherein the neurodegenerative disease is Alzheimer's disease (familial Alzheimer's disease, juvenile Alzheimer's disease, sporadic Alzheimer's disease, mild cognitive impairment etc.), cerebral amyloid angiopathy, Parkinson's disease or Down's syndrome,
(25i) the pharmaceutical according to the above-mentioned (25d), which is a prophylactic/therapeutic agent for a neurodegenerative disease,
(26) the pharmaceutical according to the above-mentioned (10), (11), (15) or (21), which is a prophylactic/therapeutic agent for a neurodegenerative disease,
(27) the diagnostic agent according to the above-mentioned (12) or (20), which is a diagnostic agent for a neurodegenerative disease,
(28) a prophylactic/therapeutic method for a neurodegenerative disease, which comprises administering to a mammal an effective amount of a compound or its salt that promotes or inhibits the activity of the protein according to the above-mentioned (1) or its partial peptide or its salt, a compound or its salt that promotes or inhibits the expression of a gene for said protein or its partial peptide or its salt, or a compound or its salt that promotes or inhibits the expression of said protein or its partial peptide or its salt,
(29) a method of inhibiting production of β-amyloid, which comprises administering to a mammal an effective amount of a compound or its salt that promotes the activity of the protein according to the above-mentioned (1) or its partial peptide or its salt, a compound or its salt that promotes the expression of a gene for said protein or its partial peptide or its salt, or a compound or its salt that promotes the expression of said protein or its partial peptide or its salt,
(30) a method of inhibiting cell-death, which comprises administering to a mammal an effective amount of a compound or its salt that inhibits the activity of the protein according to the above-mentioned (1) or its partial peptide or its salt, a compound or its salt that inhibits the expression of a gene for said protein or its partial peptide or its salt, or a compound or its salt that inhibits the expression of said protein or its partial peptide or its salt,
(31) a prophylactic/therapeutic method for a neurodegenerative disease, wherein the activity of the protein according to the above-mentioned (1) or its partial peptide or its salt is promoted or inhibited, the expression of a gene for said protein or its partial peptide or its salt is promoted or inhibited, or the expression of said protein or its partial peptide or its salt is promoted or inhibited,
(32) a method of inhibiting production of β-amyloid, wherein the activity of the protein according to the above-mentioned (1) or its partial peptide or its salt is promoted, the expression of a gene for said protein or its partial peptide or its salt is promoted, or the expression of said protein or its partial peptide or its salt is promoted,
(33) a method of inhibiting cell-death, wherein the activity of the protein according to the above-mentioned (1) or its partial peptide or its salt is inhibited, the expression of a gene for said protein or its partial peptide or its salt is inhibited, or the expression of said protein or its partial peptide or its salt is inhibited,
(34) use of a compound or its salt that promotes or inhibits the activity of the protein according to the above-mentioned (1) or its partial peptide or its salt, a compound or its salt that promotes or inhibits the expression of a gene for said protein or its partial peptide or its salt, or a compound or its salt that promotes or inhibits the expression of said protein or its partial peptide or its salt, for the manufacture of a prophylactic/therapeutic agent for a neurodegenerative disease,
(35) use of a compound or its salt that promotes the activity of the protein according to the above-mentioned (1) or its partial peptide or its salt, a compound or its salt that promotes the expression of a gene for said protein or its partial peptide or its salt, or a compound or its salt that promotes the expression of said protein or its partial peptide or its salt, for the manufacture of a β-amyloid production inhibitor, and
(36) use of a compound or its salt that inhibits the activity of the protein according to the above-mentioned (1) or its partial peptide or its salt, a compound or its salt that inhibits the expression of a gene for said protein or its partial peptide or its salt, or a compound or its salt that inhibits the expression of said protein or its partial peptide or its salt, for the manufacture of an cell-death inhibitor.

### BRIEF DESCRIPTION OF DRAWINGS

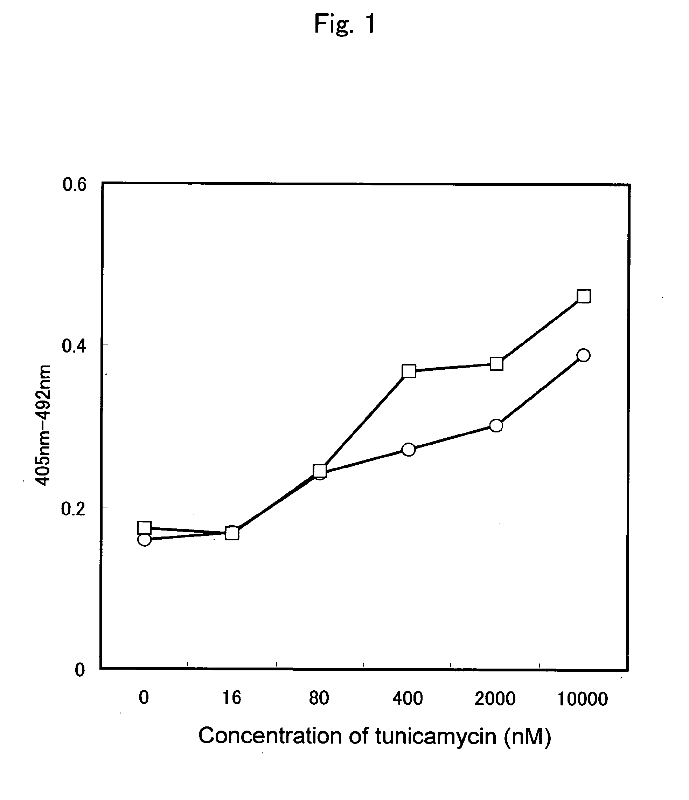

Fig. 1 shows the amount, in terms of OD405-492, of cleaved DNA in a cell after 24 hours of stimulation with tunicamycin. In the graph, absorbance is shown on the ordinate, and the concentration of tunicamycin on the abscissa. The amount of cleaved DNA in the control cell is shown in -○-, and the amount of cleaved DNA in the cell transformed with the C1 gene is shown in -□-.

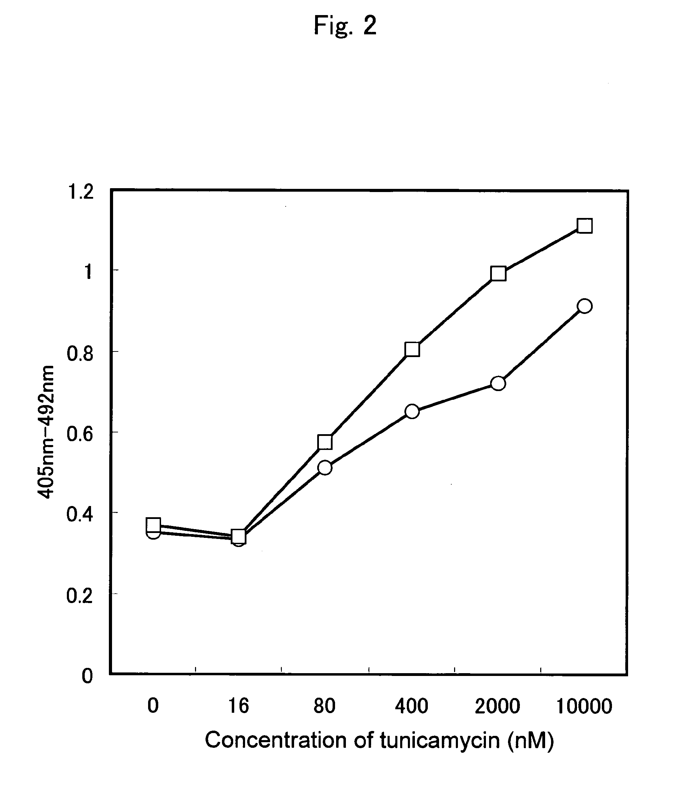

Fig. 2 shows the amount, in terms of OD405-492, of cleaved DNA in a cell after 48 hours of stimulation with tunicamycin. In the graph, absorbance is shown on the ordinate, and the concentration of tunicamycin on the abscissa. The amount of cleaved DNA in the control cell is shown in -O-, and the amount of cleaved DNA in the cell transformed with the C1 gene is shown in -□-.

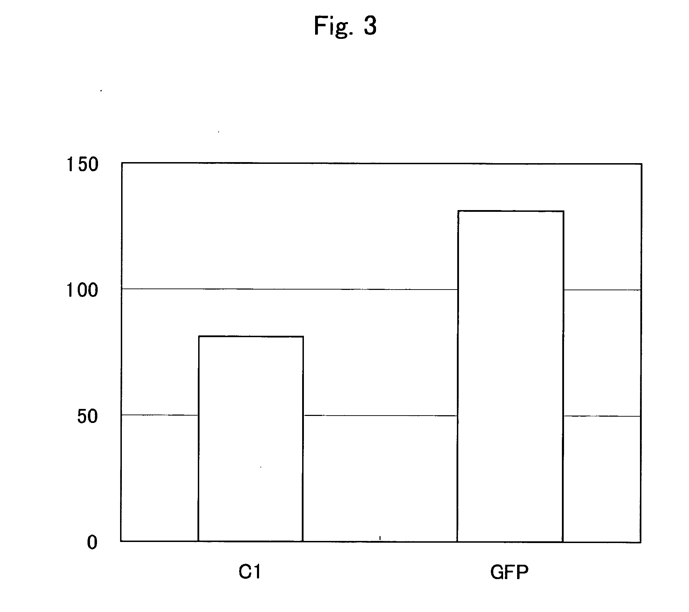

Fig. 3 shows the concentration of Aβ40 (pM) in a cell culture supernatant. In the graph, Aβ40 concentration is shown on the ordinate. On the abscissa, C1 shows the cell transformed with C1, and GFP shows IMR-32 cell transformed with GFP (control cell).

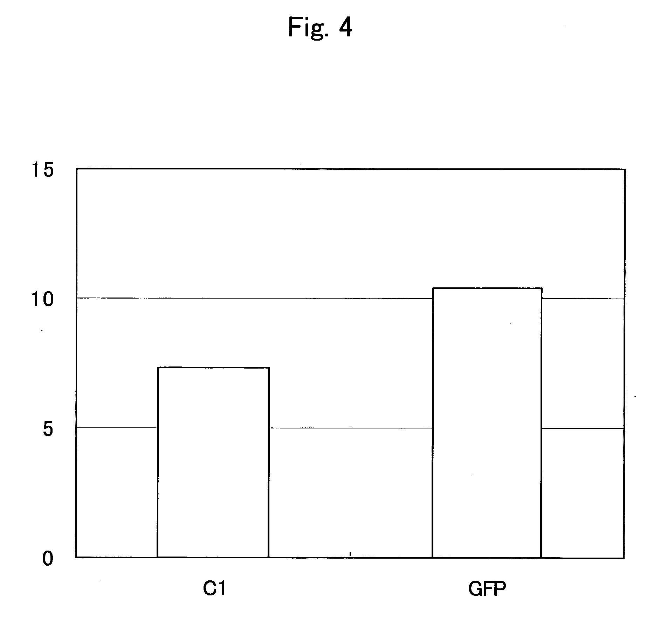

Fig. 4 shows the concentration of Aβ42 (pM) in a cell culture supernatant. In the graph, Aβ42 concentration is shown on the ordinate. On the abscissa, C1 shows the cell transformed with C1, and GFP shows IMR-32 cell transformed with GFP (control cell).

### BEST MODE FOR CARRYING OUT THE INVENTION

A protein comprising the same or substantially the same amino acid sequence as an amino acid sequence represented by SEQ ID NO: 1 (hereinafter, the protein is referred as sometimes to the protein of the present invention) may be any protein derived from any cells (e.g., liver cells, splenocytes, nerve cells, glial cells, β cells of pancreas, bone marrow cells, mesangial cells, Langerhans' cells, epidermic cells, epithelial cells, goblet cells, endothelial cells, smooth muscular cells, fibroblasts, fibrocytes, myocytes, fat cells, immune cells (e.g., macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), or any tissues where such cells are present, e.g., brain or any region of the brain (e.g., olfactory bulb, amygdaloid nucleus, basal ganglia, hippocampus, thalamus, hypothalamus, cerebral cortex, medulla oblongata and cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc. from human and non-human mammals (e.g., guinea pigs, rats, mice, chickens, rabbits, swine, sheep, bovine, monkeys, etc.), or the protein may also be a synthetic protein.

Substantially the same amino acid sequence as that represented by SEQ ID NO: 1 includes, for example, an amino acid sequence having at least about 85% homology, preferably at least about 90% homology, more preferably at least about 95% homology and further more preferably at least about 98% homology to the amino acid sequence represented by SEQ ID NO: 1.

The homology among the amino acid sequences can be calculated using homology calculation algorism NCBI BLAST (National Center for Biotechnology Information Basic Local Alignment Search Tool) under the following conditions: expected value = 10; gap is allowable; matrix = BLOSUM62; filtering = OFF.

Preferable examples of the protein comprising substantially the same amino acid sequence as that represented by SEQ ID NO: 1 include a protein comprising substantially the same amino acid sequence as that represented by SEQ ID NO: 1 and having an activity substantially equivalent to that of a protein comprising the amino acid sequence represented by SEQ ID NO: 1, etc.

The substantially equivalent activity includes, for example, an cell-death promoting activity, a β-amyloid production-inhibiting activity, etc. The term "substantially equivalent" is used to mean that the nature of the activity is the same (for example, physiologically or pharmacologically). Therefore, although it is preferred that the cell-death promoting activity be equivalent (e.g., about 0.01- to 100-fold, preferably about 0.1- to 10-fold, more preferably about 0.5- to 2-fold), quantitative factors such as a level of the activity, a molecular weight of the protein, etc. may differ.

The cell-death promoting activity can be determined according to a publicly known method, for example, by a method described in Chem. Pharm. Bull, 41, 118, 1993, or its modified method.

The β-amyloid production-inhibiting activity can be determined according to a publicly known method, for example, by a method described in Biochemistry, 24, 10272, 1995, or its modified method.

The protein of the present invention includes, for example, so called muteins, such as proteins comprising (1) (i) the amino acid sequence represented by SEQ ID NO: 1,
wherein at least 1 or 2 amino acids (for example approximately 1 to 60 amino acids, preferably approximately 1 to 30 amino acids, more preferably approximately 1 to 10 amino acids and most preferably several (1 to 5) amino acids) are deleted, (ii) the amino acid sequence represented by SEQ ID NO: 1, to which at least 1 or 2 amino acids (for example approximately 1 to 100 amino acids, preferably approximately 1 to 30 amino acids, more preferably approximately 1 to 10 amino acids and most preferably several (1 to 5) amino acids) are added, (iii) the amino acid sequence represented by SEQ ID NO: 1, into which at least 1 or 2 amino acids (for example approximately 1 to 100 amino acids, preferably approximately 1 to 30 amino acids, more preferably approximately 1 to 10 amino acids and most preferably several (1 to 5) amino acids) are inserted, (iv) the amino acid sequence represented by SEQ ID NO: 1, wherein at least 1 or 2 amino acids (for example approximately 1 to 100 amino acids, preferably approximately 1 to 30 amino acids, more preferably approximately 1 to 10 amino acids and most preferably several (1 to 5) amino acids) are substituted by other amino acids or (v) an amino acid sequence which is a combination of these sequences.

When the amino acid sequence has undergone insertion, deletion or substitution as described above, the position of the insertion, deletion or substitution is not particularly limited.

The proteins in the present specification are represented in accordance with the conventional way of describing peptides, that is, the N-terminus (amino terminus) at the left hand and the C-terminus (carboxyl terminus) at the right hand. In the proteins of the present invention including the protein comprising the amino acid sequence represented by SEQ ID NO: 1, the C-terminus may be either a carboxyl group (-COOH), a carboxylate (-COO⁻), an amide (-CONH₂) or an ester (-COOR).

The ester group shown by R include, for example, a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, α-naphthyl, etc.; a C₇₋₁₄ aralkyl group such as a phenyl-C₁₋₂-alkyl group, e.g., benzyl, phenethyl, etc., or an α-naphthyl-C₁₋₂-alkyl group such as α-naphthylmethyl, etc.; and a pivalivaloyloxymethyl group.

When the protein of the present invention has a carboxyl group (or a carboxylate) at a position other than the C-terminus, the carboxyl group may be amidated or esterified, and such an amide or ester is also included within the protein of the present invention. The ester group may be the same group as that described with respect to the C-terminus described above.

Furthermore, examples of the protein of the present invention include variants of the above proteins, wherein the N-terminal amino acid residue (for example, a methionine residue) of the protein supra is protected with a protecting group (for example, a C₁₋₆ acyl group such as a C₁₋₆ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins bound to sugar chains.

Specific examples of the protein of the present invention include, for example, a protein comprising the amino acid sequence represented by SEQ ID NO: 1.

Partial peptides of the protein of the present invention may be any partial peptides of the protein of the present invention described above, and for example, those having properties similar to those of the protein of the present invention described above.

Specifically, for the purpose of preparing the antibody of the present invention described later, peptides having an amino acid sequence in positions 1 to 30 or 234 to 264 in the amino acid sequence represented by SEQ ID NO: 1 are used. For example, peptides having at least 20, preferably at least 50, more preferably at least 70, still more preferably at least 100 and most preferably at least 200 amino acids in the amino acid sequence which constitutes the protein of the present invention are used.

The partial peptide of the present invention includes partial peptides of the amino acid sequence described above, wherein 1 or more amino acids (preferably about 1 to 10 amino acids, more preferably several (1 to 5) amino acids) may be deleted; to which 1 or more amino acids (preferably about 1 to 20 amino acids, more preferably about 1 to 10 amino acids, still more preferably several (1 to 5) amino acids) may be added; into which 1 or more amino acids (preferably about 1 to 20 amino acids, more preferably 1 to 10 amino acids, still more preferably about 1 to 5 amino acids) may be inserted; or in which 1 or more amino acids (preferably about 1 to 10 amino acids, more preferably several amino acids, still more preferably about 1 to 5 amino acids) may be substituted by other amino acids.

In the partial peptide of the present invention, the C-terminus may be either a carboxyl group (-COOH), a carboxylate (-COO⁻), an amide (-CONH₂) or an ester (-COOR).

The partial peptide of the present invention, similar to the protein of the present invention described above, includes those having a carboxyl group (or a carboxylate) at a position other than the C-terminus, those wherein an amino group of the N-terminal amino acid residue (e.g., methionine residue) is protected with a protecting group, those wherein the N-terminal region is cleaved in vivo and a glutamine reissue thus formed is pyroglutaminated, those wherein a substituent on the side chain of an amino acid in the molecule is protected with a suitable protecting group, or conjugated proteins such as glycoproteins having sugar chains bound thereto.

The partial peptide of the present invention can also be used as an antigen for preparing an antibody.

As salts of the protein or partial peptide of the present invention, use is made of salts with physiologically acceptable acids (e.g., inorganic acids or organic acids) or bases (e.g., alkali metal salts), preferably physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein or partial peptide of the present invention or salts thereof may be manufactured from the human and other warm-blooded animal cells or tissues described above by a publicly known protein purification method, or by culturing a transformant that comprises the DNA encoding the protein of the present invention. Furthermore, the protein or partial peptide or salts thereof may also be manufactured by a peptide synthesis method which will be described below.

When the protein of the present invention is manufactured from human and non-human mammalian tissues or cells, the human or non-human mammalian tissues or cells are homogenized, then extracted with an acid or the like, and the extract is isolated and purified by a combination of chromatographic techniques such as reverse phase chromatography, ion exchange chromatography, and the like.

To synthesize the protein of the present invention, its partial peptide, or salts or amides thereof according to the present invention, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein according to various condensation methods publicly known in the art. At the end of the reaction, the protein is cut out from the resin and at the same time, the protecting groups are removed. Then, intramolecular disulfide bond-forming reaction is performed in a highly diluted solution to obtain the objective protein or partial peptide or its amides.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse effect on the subsequent reaction.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group such as acetyl group, an aroyl group such as benzoyl group, and a group derived from carbonic acid, such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids in which the amino groups are activated in the starting material, the corresponding phosphoric amides are employed.

To eliminate (split off) the protecting groups, there are used catalytic reduction under hydrogen gas flow in the presence of a catalyst such as Pd-black or Pd-carbon; acid treatment with anhydrous hydrogen fluoride, methanesulfonic acid, trifluoromethanesulfonic acid or trifluoroacetic acid, or a mixture solution of these acids; treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group used as the protecting group for the imidazole of histidine is removed by treatment with thiophenol. Formyl group used as the protecting group of the indole of tryptophan is eliminated by the aforesaid acid treatment in the presence of 1,2-ethanedithiol or 1,4-butanedithiol, as well as by treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In an alternative method for obtaining the amides of the protein or partial peptide of the present invention, for example, the α-carboxyl group of the carboxy terminal amino acid is first protected by amidation, and the peptide (protein) chain is then extended from the amino group side to a desired length. Thereafter, a protein or partial peptide in which only the protecting group of the N-terminal α-amino group in the peptide chain has been eliminated from the protein and a protein or partial peptide in which only the protecting group of the C-terminal carboxyl group has been eliminated are prepared. These proteins or peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the ester form of the desired protein or peptide.

The partial peptide of the present invention or its salts can be manufactured by publicly known methods for peptide synthesis, or by cleaving the protein of the present invention with a suitable peptidase. For the methods for peptide synthesis, for example, either solid phase synthesis or liquid phase synthesis may be used. That is, the partial peptide or amino acids that can construct the partial peptide of the present invention are condensed with the remaining part. Where the product has protecting groups, these protecting groups are removed to give the desired peptide. Publicly known methods for condensation and elimination of the protecting groups are described for example in (i) to (v) below.
(i) M. Bodanszky & M.A. Ondetti: Peptide Synthesis, Interscience Publishers, New York (1966)
(ii) Schroeder & Luebke: The Peptide, Academic Press, New York (1965)
(iii) Nobuo Izumiya, et al.: Peptide Gosei-no-Kiso to Jikken (Basics and experiments of peptide synthesis), published by Maruzen Co. (1975)
(iv) Haruaki Yajima & Shunpei Sakakibara: Seikagaku Jikken Koza (Biochemical Experiment) 1, Tanpakushitsu no Kagaku (Chemistry of Proteins) IV, 205 (1977)
(v) Haruaki Yajima, ed.: Zoku Iyakuhin no Kaihatsu (A sequel to Development of Pharmaceuticals), Vol. 14, Peptide Synthesis, published by Hirokawa Shoten

After completion of the reaction, the product may be isolated and purified by a combination of conventional purification methods such as solvent extraction, distillation, column chromatography, liquid chromatography and recrystallization to give the partial peptide of the present invention. When the partial peptide obtained by the above methods is in a free form, the partial peptide can be converted into an appropriate salt by a publicly known method or a modification thereof; when the partial peptide is obtained in a salt form, it can be converted into a free form or another salt by a publicly known method or a modification thereof.

The polynucleotide encoding the protein of the present invention may be any polynucleotide so long as it comprises the nucleotide sequence encoding the protein of the present invention described above. The polynucleotide is preferably DNA. The DNA may also be any of genomic DNA, genomic cDNA library, cDNA derived from the cells or tissues described above, cDNA library derived from the cells or tissues described above, and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

The DNA encoding the protein of the present invention may be any DNA insofar as it is DNA comprising the nucleotide sequence represented by, for example, SEQ ID NO: 2, or DNA hybridizing under high stringent conditions with the nucleotide sequence represented by SEQ ID NO: 2 and encoding a protein having an activity substantially equivalent to that of a protein comprising the amino acid sequence represented by SEQ ID NO: 1.

Specific examples of the DNA hybridizable under high stringent conditions with the nucleotide sequence represented by SEQ ID NO: 2 include DNA comprising a nucleotide sequence having at least about 85% homology, preferably at least about 90% homology, more preferably at least about 95% homology and most preferably at least about 98% to the nucleotide sequence represented by SEQ ID NO: 2.

The homology among the nucleotide sequences can be calculated using homology calculation algorism NCBI BLAST (National Center for Biotechnology Information Basic Local Alignment Search Tool) under the following conditions: expected value = 10; gap is allowable; filtering = ON; match score =1; mismatch score =-3.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at about 19 mM to about 40 mM, preferably about 19 mM to about 20 mM at a temperature of about 50°C to about 70°C, preferably about 60°C to about 65°C. In particular, hybridization conditions in a sodium concentration of about 19 mM at a temperature of about 65°C are most preferred.

More specifically, DNA comprising the nucleotide sequence represented by SEQ ID NO: 2 or the like is used as the DNA encoding a protein comprising the amino acid sequence represented by SEQ ID NO: 1.

The polynucleotide (for example, DNA) encoding the partial peptide of the present invention may be any polynucleotide so long as it comprises a nucleotide sequence encoding the partial peptide of the present invention described above. The DNA may also be any of genomic DNA, genomic DNA library, cDNA derived from the cells and tissues described above, cDNA library derived from the cells and tissues described above and synthetic DNA.

As the DNA encoding the partial peptide of the present invention, use is made of DNA comprising a part of DNA comprising the nucleotide sequence represented by, for example, SEQ ID NO: 2, or DNA comprising a part of DNA hybridizing under high stringent conditions with the nucleotide sequence represented by SEQ ID NO: 2 and encoding a protein having an activity substantially equivalent to that of the protein of the present invention.

The DNA hybridizable with the nucleotide sequence represented by SEQ ID NO: 2 has the same meaning as described above.

As the hybridization method and high stringent conditions, those described above are used.

For cloning of the DNA that completely encodes the protein of the present invention or its partial peptide (hereinafter sometimes collectively referred to as the protein of the present invention in the description of cloning and expression of the DNA encoding the same), the DNA may be amplified by PCR using synthetic DNA primers comprising a part of the nucleotide sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the nucleotide sequence of the DNA can be effected by publicly known methods such as the ODA-LA PCR method, the Gapped duplex method or the Kunkel method or its modification by using PCR, or a publicly known kit available as Mutant™-super Express Km or Mutan™-K (both manufactured by Takara Shuzo Co., Ltd.).

The cloned DNA encoding the protein can be used as it is, depending upon purpose or if desired after digestion with a restriction enzyme or after addition of a linker thereto. The DNA may have ATG as a translation initiation codon at the 5' end thereof and may further have TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA encoding the protein of the present invention, and then (b) ligating the DNA fragment with an appropriate expression vector downstream from a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, λP_{L} promoter, lpp promoter, T7 promoter etc. In the case of using bacteria of the genus Bacillus as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in dhfr gene-deficient Chinese hamster's cells, selection can also be made on thymidine free media.

If necessary and desired, a signal sequence that matches with a host is added to the N-terminus of the receptor protein of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector comprising the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977)).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982).

Bacteria belonging to the genus Bacillus can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55 (1988), etc.

Animal cells can be transformed, for example, according to the method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Thus, the transformant transformed with the expression vector comprising the DNA encoding the protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for cultivating the bacteria belonging to the genus Escherichia is M9 medium supplemented with glucose and Casamino acids (Journal of Experiments in Molecular Genetics, 431-433, Cold Spring Harbor Laboratory, New York, 1972). If necessary and desired, a chemical such as 3β-indolylacrylic acid can be added to the medium to activate the promoter efficiently.

Where the bacteria belonging to the genus Escherichia are used as the host, the transformant is usually cultivated at about 15°C to about 43°C for about 3 hours to about 24 hours. If necessary and desired, the culture may be aerated or agitated.

Where the bacteria belonging to the genus Bacillus are used as the host, the transformant is cultivated generally at about 30°C to about 40°C for about 6 hours to about 24 hours. If necessary and desired, the culture can be aerated or agitated.

Where yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

Where insect cells or insects are used as the host, the transformant is cultivated in, for example, Grace's Insect Medium (Nature, 195, 788 (1962)) to which an appropriate additive such as inactivated 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the protein of the present invention can be produced in the cell, in the cell membrane or out of the cell of the transformant.

The protein of the present invention can be separated and purified from the culture described above by the following procedures.

When the protein of the present invention is extracted from the culture or cells after cultivation, the transformants or cells are collected by a publicly known method and suspended in an appropriate buffer. The transformants or cells are then disrupted by publicly known methods such as ultrasonication, treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™, etc. When the protein is secreted in the culture, the supernatant after completion of the cultivation can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing a difference in hydrophobicity such as reverse phase high performance liquid chromatography, etc.; a method utilizing a difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The presence of the thus formed protein of the present invention can be determined by an enzyme immunoassay or Western blotting using a specific antibody.

Antibodies to the protein or partial peptide of the present invention or salts thereof may be any of polyclonal antibodies and monoclonal antibodies as long as they are capable of recognizing the protein or partial peptide of the present invention or salts thereof.

The antibodies to the protein or partial peptide of the present invention or salts thereof (sometimes simply referred to as the protein of the present invention) may be manufactured according to publicly known methods for manufacturing antibodies or antisera by using the protein of the present invention as an antigen.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable warm-blooded animals are monkeys, rabbits, dogs, guinea pigs, mice, rats, sheep, goats and chickens, with mice and rats being preferred.

In the preparation of monoclonal antibody-producing cells, warm-blooded animals, e.g., mice, immunized with an antigen wherein the antibody titer is noted are selected, then the spleen or lymph node is collected 2 to 5 days after the final immunization and antibody-producing cells contained therein are fused with myeloma cells from an animal of the same or different species to give monoclonal antibody-producing hybridomas. Measurement of the antibody titer in antisera may be conducted, for example, by reacting a labeled form of the protein which will be described later, with the antiserum followed by assaying the binding activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., among which PEG is preferably employed.

Examples of the myeloma cells used include myeloma cells of warm-blooded animals, such as NS-1, P3U1, SP2/0, AP-1 etc., among which P3U1 is particularly preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubation at about 20 to about 40°C, preferably at about 30 to about 37°C for about 1 to about 10 minutes, efficient cell fusion can be carried out.

Various methods can be used for screening of a monoclonal antibody-producing hybridoma. Examples of such methods include a method which comprises adding the supernatant of hybridoma to a solid phase (e.g., microplate) adsorbed with the protein as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum-free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% CO₂. The antibody titer of the culture supernatant of hybridomas can be determined as in the assay for the antibody titer in antisera described above.

### (b) Purification of monoclonal antibody

Separation and purification of a monoclonal antibody can be carried out by the conventional methods for separation and purification of immunoglobulins [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, an immunogen (protein antigen) itself or a complex prepared from the immunogen and a carrier protein is used to immunize a warm-blooded animal in a manner similar to the method described above for the manufacture of monoclonal antibodies. The product containing the antibody to the protein of the present invention is collected from the immunized animal, followed by separation and purification of the antibody.

In the complex of an immunogen and a carrier protein used to immunize a warm-blooded animal, the type of carrier protein and the mixing ratio of a carrier to hapten may be any type and in any ratio, as long as the antibody is efficiently produced against the hapten immunized by crosslinking to the carrier. For example, bovine serum albumin, bovine thyroglobulins, hemocyanin or the like is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide-activated ester, activated ester reagents containing thiol group or dithiopyridyl group, etc. are used for the coupling.

The condensation product is administered to warm-blooded animals either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of warm-blooded animals immunized by the method described above.

The polyclonal antibody titer in antiserum can be assayed by the same procedure as that for the determination of antibody titer in antiserum described above. The separation and purification of the polyclonal antibody can be carried out according to the method for the separation and purification of immunoglobulins performed as applied to the separation and purification of monoclonal antibodies described hereinabove.

The antisense polynucleotide comprising a complementary or substantially complementary nucleotide sequence, or a part of thereof, to the nucleotide sequence of a polynucleotide encoding the protein or partial peptide of the present invention (which in the following description of the antisense polynucleotide, is referred to sometimes as the DNA of the present invention) can be any antisense polynucleotide so long as it comprises a complementary or substantially complementary nucleotide sequence, or a part thereof, to that of the DNA of the present invention and capable of suppressing expression of the DNA. The antisense polynucleotide is preferably antisense DNA.

The nucleotide sequence substantially complementary to the DNA of the present invention includes, for example, a nucleotide sequence having at least about 70% homology, preferably at least about 80% homology, more preferably at least about 90% homology and most preferably at least about 95% homology, to the full-length nucleotide sequence or partial nucleotide sequence of the nucleotide sequence complementary to the DNA of the present invention (i.e., complementary strand to the DNA of the present invention). Particularly, (A) an antisense polynucleotide directed to translational inhibition is preferably an antisense polynucleotide having at least about 70% homology, preferably at least about 80% homology, more preferably at least about 90% homology and most preferably at least about 95% homology, to the complementary strand of the nucleotide sequence encoding the N-terminal site of the protein of the present invention (e.g., the nucleotide sequence around the initiation codon), in the entire nucleotide sequence of the complementary strand to the DNA of the present invention, and (B) an antisense polynucleotide directed to RNA decomposition by RNase H is preferably an antisense polynucleotide having at least about 70% homology, preferably at least about 80% homology, more preferably at least about 90% homology and most preferably at least about 95% homology, to the complementary strand of the entire nucleotide sequence of the intron-containing DNA of the present invention.

Specifically, the nucleotide sequence substantially complementary to the DNA of the present invention is an antisense polynucleotide having a complementary or substantially complementary nucleotide sequence, or a part thereof, to the nucleotide sequence of DNA comprising the nucleotide sequence represented by SEQ ID NO: 2, preferably an antisense polynucleotide having a complementary or substantially complementary nucleotide sequence, or a part thereof, to the nucleotide sequence of DNA comprising the nucleotide sequence represented by SEQ ID NO: 2 (more preferably, an antisense polynucleotide having a nucleotide sequence complementary to the nucleotide sequence of DNA comprising the nucleotide sequence represented by SEQ ID NO: 2, or an antisense polynucleotide having a part thereof).

The antisense polynucleotide is composed of usually about 10 to 40 bases, preferably about 15 to 30 bases.

For preventing degradation by hydrolases such as nuclease etc., phosphoric acid residues (phosphates) of nucleotides constituting the antisense DNA may be substituted by chemically modified phosphoric acid residues such as phosphorothioate, methylphosphonate, phosphorodithionate etc. A sugar (deoxy ribose) of each nucleotide may be substituted by a chemically modified sugar structure such as a 2'-O-methylated structure, and a base moiety (pyrimidine, purine) of each nucleotide may have undergone chemical modification, and the antisense polynucleotide is not limited insofar as it hybridizes with DNA having the nucleotide sequence represented by SEQ ID NO: 2. These antisense polynucleotides can be produced by a known DNA synthesizer etc.

According to the present invention, antisense polynucleotides capable of inhibiting the replication or expression of the gene for the protein of the present invention can be designed and synthesized based on the nucleotide sequence information of the cloned or determined DNA encoding the protein. Such nucleotide (nucleic acid) is capable of hybridizing with RNA of the protein gene for the present invention to inhibit the synthesis or function of said RNA, or is capable of modulating or controlling the expression of the protein gene for the invention via interaction with the protein-associated RNA of the invention. Polynucleotides complementary to the selected sequences of the protein-associated RNA of the invention, and polynucleotides specifically hybridizable with the protein-associated RNA of the invention, are useful in modulating or controlling the expression of the protein gene for the invention in vivo and in vitro, and useful for the treatment or diagnosis of diseases. The term "corresponding" is used to mean homologous to or complementary to a particular sequence of the nucleotide, nucleotide sequence or nucleic acid including the gene. The term "corresponding" between nucleotides, nucleotide sequences or nucleic acids and proteins usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to, and hybridizable with, at least a part of the target region, can be denoted to be "antisense" to the polynucleotides in the target region. Examples of the antisense polynucleotides include polydeoxyribonucleotides containing 2-deoxy-D-ribose, polyribonucleotides containing D-ribose, any other type of polynucleotides which are N-glycosides of a purine or pyrimidine base, or other polymers having non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA: RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., α anomeric nucleic acids, etc.), and the like. Herein the terms "nucleoside", "nucleotide" and "nucleic acid" are used to refer to moieties that contain not only the purine and pyrimidine bases, but also other heterocyclic bases which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide of the present invention is RNA, DNA or a modified nucleic acid (RNA, DNA). Specific examples of the modified nucleic acid include, but are not limited to, sulfur and thiophosphate derivatives of nucleic acids and those resistant to degradation of polynucleoside amides or oligonucleoside amides. The antisense polynucleotides of the present invention can be modified preferably based on the following design, that is, by increasing the intracellular stability of the antisense polynucleotide, increasing the cell permeability of the antisense polynucleotide, increasing the affinity of the antisense polynucleotide for the targeted sense strand to a higher level, or minimizing the toxicity, if any, of the antisense polynucleotide. Many of such modifications are known in the art, as disclosed in Pharm. Tech. Japan, Vol. 8, p. 247 or 395, 1992; Antisense Research and Applications, CRC Press, 1993; etc.

The antisense polynucleotide of the present invention may contain altered or modified sugars, bases or linkages, may also be provided in a specialized form such as liposomes or microspheres, may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the polynucleotide at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense polynucleotide can be examined using the transformant of the present invention, the gene expression system of the present invention in vivo and in vitro, or a translation system of the protein of the present invention in vivo and in vitro.

Hereinafter, the protein of the present invention, its partial peptides, or salts thereof (hereinafter sometimes referred to as the protein of the present invention), the polynucleotide encoding the protein of the present invention or its partial peptides (hereinafter sometimes referred to as the DNA of the present invention), the antibodies to the protein of the present invention, its partial peptides, or salts thereof (hereinafter sometimes referred to as the antibodies of the present invention) and the antisense polynucleotide of the DNA of the present invention (hereinafter sometimes referred to as the antisense polynucleotide of the present invention) are specifically described for the use or applications.

The protein of the present invention can be used as a disease marker because its expression is increased upon application of endoplasmic reticulum stress to nerve cells. For example, it is useful as a marker for early diagnosis of nerve degeneration, judgment of the severeness of a symptom, or prediction of disease progress. Accordingly, a pharmaceutical comprising an antisense polynucleotide of a gene encoding the protein of the present invention, a compound or its salt inhibiting the activity of the protein of the present invention, or an antibody against to the protein of the present invention can be used as a low-toxic prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

### (1) Prophylactic /therapeutic agent for diseases associated with the protein of the present invention

The protein of the present invention regulates repair and decomposition of abnormal proteins, neuronal death, amyloid production, etc. because its expression is increased upon application of endoplasmic reticulum stress to nerve cells.

Therefore, where the DNA encoding the protein of the present invention is abnormal or deficient, or where the expression level of the protein of the present invention is reduced, there occur a variety of diseases, for example, neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

Accordingly, the protein of the present invention and the DNA of the present invention can be used as safe pharmaceuticals such as prophylactic/therapeutic agents for diseases such as neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.). The protein of the present invention and the DNA of the present invention can also be used as a β-amyloid production inhibitor etc.

For example, when there is a patient who cannot sufficiently or normally exhibit the activity of the protein of the present invention because of a decrease or deficiency in the protein of the present invention in the living body, (a) DNA encoding the protein of the invention is administered into the patient to express the protein of the invention in the living body, (b) the DNA is inserted into target cells to express the protein of the invention and the cells are transplanted to the patient, or (c) the protein of the invention is administered into the patient, whereby the role of the protein of the invention can be exhibited sufficiently or normally in the patient.

When the DNA of the present invention is used as the prophylactic/therapeutic agents described above, the DNA itself is administered; alternatively, the DNA is inserted into an appropriate vector such as retrovirus vector, adenovirus vector, adenovirus-associated virus vector, etc. and then administered into humans or warm-blooded animals in a conventional manner. The DNA of the present invention may also be administered as naked DNA, or in the form of a preparation prepared with physiologically acceptable carriers such as adjuvants to assist its uptake by gene gun or through a catheter such as a catheter with a hydrogel.

When the protein of the present invention is used as the aforesaid prophylactic/therapeutic agent, the protein is advantageously used on a purified level of at least 90%, preferably at least 95%, more preferably at least 98% and most preferably at least 99%.

For example, the protein of the present invention can be used orally in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be manufactured for example by mixing the protein of the present invention with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin, alginic acid, etc., a lubricant such as magnesium stearate, a sweetening agent such as sucrose, lactose or saccharin, and a flavoring agent such as peppermint, akamono oil or cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredient in a vehicle such as water for injection, with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate solubilizer such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil, soybean oil, etc., which may also be used in combination with a solubilizer such as benzyl benzoate, benzyl alcohol, etc. The prophylactic/therapeutic agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus prepared liquid for injection is normally filled in an appropriate ampoule.

The vector in which the DNA of the present invention is inserted may also be prepared into pharmaceutical preparations in a manner similar to the procedures above, and such preparations are generally used parenterally.

Since the thus obtained pharmaceutical preparation is safe and low toxic, and can be administered to, for example, warm-blooded animals (e.g., human, rat, mouse, guinea pig, rabbit, chicken, sheep, swine, bovine, horse, cat, dog, monkey, chimpanzee etc.).

The dose of such as the protein of the present invention may vary depending on target disease, subject of administration, route for administration, etc. When the protein of the present invention is orally administered for example for the purpose of treatment of Alzheimer's disease, the protein is administered to adult (as 60 kg) generally in a daily dose of approximately 0.1 mg to 100 mg, preferably approximately 1.0 mg to 50 mg, more preferably approximately 1.0 to 20 mg. When the protein is parenterally administered, a single dose of the protein may vary depending on subject of administration, target disease, etc. When the protein is administered in the form of an injection to adult (as 60 kg) for the purpose of treatment ofAlzheimer's disease, it is advantageous to inject the protein at the affectedlesion generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

### (2) Screening of a candidate compounds for diseases

The protein of the present invention regulates repair and decomposition of abnormal proteins, neuronal death, amyloid production, etc. because its expression is increased upon application of endoplasmic reticulum stress to nerve cells. Accordingly, a compound or its salt that regulates (promotes or inhibits) the activities (for example, an cell-death promoting activity, β-amyloid production-inhibiting activity etc.) of the protein of the present invention is useful as a prophylactic/therapeutic agent for diseases, for example neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), a β-amyloid production inhibitor, an cell-death inhibitor, etc.

Accordingly, the protein of the present invention is useful as a reagent for screening a compound or its salt that regulates the activity of the protein of the present invention.

That is, the present invention provides a method of screening a compound or its salt that regulates (promotes or inhibits) the activity of the protein of the present invention, which comprises using the protein of the present invention.

Specifically, mention is made of a method of screening a promoter or an inhibitor (preferably the inhibitor), which comprises comparing (i) the cell-death promoting activity of a cell having an ability to produce the protein of the present invention with (ii) the cell-death promoting activity of a cell having an ability to produce the protein of the present invention in the presence of a test compound.

The cell-death promoting activity can be determined according to a publicly known method, for example, by a method described in Chem. Pharm. Bull, 41, 118, 1993, or its modified method.

For example, the respiration ability of a living cell is measured by using the reduction reaction of a tetrazolium salt into formazan by an intracellular dehydrogenase, and the respiration ability in the case (i) is compared with that in the case (ii) by using the absorbance of formed formazan as an indicator.

Alternatively, use is also made of a method of screening a promoter or an inhibitor (preferably the promoter), which comprises comparing (I') the β-amyloid production-inhibiting activity of a cell having an ability to produce the protein of the present invention with (ii') the β-amyloid production-inhibiting activity of a cell having an ability to produce the protein of the present invention in the presence of a test compound.

The β-amyloid production-inhibiting activity can be determined according to a publicly known method, for example, by a method described in Biochemistry, 24, 10272, 1995, or its modified method.

For example, the amount of β-amyloid in the cell culture supernatant is measured by sandwich ELISA, and the amount of β-amyloid in the case (i') is compared with that in the case (ii').

Examples of the test compounds include peptides, proteins, non-peptide compounds, synthetic compounds, fermentation products, cell extracts, plant extracts, animal tissue extracts, etc. and these compounds may be novel compounds or publicly known compounds.

To perform the screening method described above, a protein preparation is prepared by suspending cells having an ability to produce the protein of the present invention, in a buffer appropriate for screening. A buffer such as a phosphate buffer or a borate buffer having a pH value of about 4 to 10 (preferably pH of about 6 to 8) can be used so long as it does not inhibit the activity of the protein of the present invention.

As the cells having an ability to produce the protein of the present invention, for example a host (transformant) transformed with a vector comprising the DNA encoding the protein of the present invention described above is used. As the host, animal cells such as CHO cells are preferably used. In the screening, for example a transformant expressing the protein of the present invention on a cell membrane by culturing it by the method described above is preferably used.

For example, a test compound by which the activity in the above case (ii) or (ii') is increased by at least about 20%, preferably at least about 30%, more preferably at least about 50%, as compared with the activity in the above case (i) or (i') can be selected as a compound that promotes the activity of the protein of the present invention, while a test compound by which the activity in the above case (ii) or (ii') is decreased by at least about 20%, preferably at least about 30%, more preferably at least about 50%, as compared with the activity in the above case (i) or (i') can be selected as a compound that inhibits the activity of the protein of the present invention.

The compounds or salts thereof, which are obtainable using the screening method or screening kit of the present invention, are compounds selected from, e.g., peptides, proteins, non-peptide compounds, synthetic compounds, fermentation products, cell extracts, plant extracts, animal tissue extracts, blood plasma, and the like. As salts of these compounds, the same salts as those given above for the peptide of the present invention may be used.

Since expression of the gene for the protein of the present invention is increased upon application of endoplasmic reticulum stress to nerve cells, a compound or its salt that regulates (promotes or inhibits) the expression of the gene encoding the protein of the present invention can be used as a prophylactic/therapeutic agent for diseases, for example, neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), a β-amyloid production inhibitor, an cell-death inhibitor, etc.

Accordingly, the polynucleotide (for example, DNA) of the present invention is useful as a reagent for screening a compound or its salt that regulates the expression of the gene encoding the protein of the present invention.

As the screening method, mention is made of a screening method which comprises comparing the case (iii) where a cell having an ability to produce the protein of the present invention with the case (iv) where a cell having an ability to produce the protein of the present invention in the presence of a test compound.

In the above method, the expression level of the gene (specifically, the amount of the protein of the present invention or the amount of mRNA encoding the protein) is measured, and the expression level in the case (iii) is compared with that in the case (iv).

The test compound and the cell having an ability to produce the protein of the present invention include the same specific examples as described above.

The amount of the protein can determined by measuring the protein present in a cell extract according to publicly known methods, for example, Western analysis, ELISA, or a modification of the known methods, by using the antibody recognizing the protein of the present invention.

The amount of the mRNA can determined by known methods, for example Northern hybridization using SEQ ID NO: 2 or a nucleic acid containing a part thereof as a probe, or PCR using SEQ ID NO: 2 or a nucleic acid containing a part thereof as a primer, or a modification of the known methods.

For example, a test compound by which the expression level of the gene in the case (iv) is increased by at least about 20%, preferably at least about 30%, more preferably at least about 50%, as compared with the expression level in the case (iii), can be selected as a compound that promotes the expression level of the gene encoding the protein of the present invention, while a test compound by which the expression level of the gene in the case (iv) is decreased by at least about 20%, preferably at least about 30%, more preferably at least about 50%, as compared with the expression level in the case (iii), can be selected as a compound that inhibits the expression of the gene encoding the protein of the present invention.

The screening kit of the present invention comprises the protein or partial peptide of the present invention or a salt thereof, or a cell having an ability to produce the protein or partial peptide of the present invention.

The compounds or salts thereof, which are obtainable using the screening method or screening kit of the present invention, are the test compounds described above, for example compounds or salts thereof selected from, e.g., peptides, proteins, non-peptide compounds, synthetic compounds, fermentation products, cell extracts, plant extracts, animal tissue extracts, blood plasma, and the like, and compounds or salts thereof regulating the activity (for example, an cell-death promoting activity) of the protein of the present invention, or compounds or salts thereof regulating the expression of the gene for the protein of the present invention.

As salts of these compounds, the same salts as those given above for the protein of the present invention may be used.

The compound or its salt that promotes the activity of the protein of the present invention is useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), a β-amyloid production inhibitor, etc. The neurodegenerative diseases are preferably Alzheimer's disease (for example, familial Alzheimer's disease, juvenile Alzheimer's disease, sporadic Alzheimer's disease, mild cognitive impairment etc.), cerebral amyloid angiopathy, Parkinson's disease, Down's syndrome, etc.

The compound or its salt that inhibits the activity of the protein of the present invention is useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), an cell-death inhibitor, etc.

The compound or its salt that promotes the expression of a gene encoding the protein of the present invention is useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), a β-amyloid production inhibitor, etc. The neurodegenerative diseases are preferably Alzheimer's disease (for example, familial Alzheimer's disease, juvenile Alzheimer's disease, sporadic Alzheimer's disease, mild cognitive impairment etc.), cerebral amyloid angiopathy, Parkinson's disease, Down's syndrome, etc.

The compound or its salt that inhibits the expression of a gene encoding the protein of the present invention is useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), an cell-death inhibitor, etc.

When the compound or its salt obtained by the screening method or screening kit of the present invention is used as the prophylactic/therapeutic agents supra, the compound or its salt can be prepared into a pharmaceutical composition in a conventional manner.

Examples of the composition for oral administration include solid or liquid preparations, specifically tablets (including dragees and film-coated tablets), pills, granules, powdery preparations, capsules (including soft capsules), syrup, emulsions, suspensions, etc. Such a composition is manufactured by publicly known methods and contains a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular, drip and intra-joint injections. Such injections are prepared by publicly known methods, e.g., by dissolving, suspending or emulsifying the aforesaid antibody or its salts in a sterile aqueous or oily liquid medium used usually in injections. For the aqueous medium for injection, for example, physiological saline and isotonic solutions containing glucose and other adjuvant, etc. are used. Appropriate dissolution aids, for example, an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. may be used in combination. For the oily solution, for example, sesame oil, soybean oil, etc. are used, and dissolution aids such as benzyl benzoate, benzyl alcohol, etc. may be used in combination. The thus prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the compound described above is contained generally in a dose of approximately 5 to 500 mg per unit dosage form, approximately 5 to 100 mg especially for injections and approximately 10 to 250 mg for other preparations.

Each composition described above may further contain other active components unless formulation with the compound causes any adverse interaction.

Since the thus obtained pharmaceutical preparation is safe and low toxic, and can be administered orally or parenterally to, for example, humans or warm-blooded animals (e.g., mouse, rat, rabbit, sheep, swine, bovine, horse, chicken, cat, dog, monkey, chimpanzee etc.).

The dose of the compound of the present invention or its salt may vary depending on its action, target disease, subject of administration, route for administration, etc. When the compound of the present invention or its salt that regulates (e.g., promotes) the activity of the protein of the present invention is orally administered for example for the purpose of treatment of Alzheimer's disease, the compound or its salt is administered to adult (as 60 kg) generally in a daily dose of approximately 0.1 mg to 100 mg, preferably approximately 1.0 mg to 50 mg, more preferably approximately 1.0 to 20 mg. When the compound or its salt is parenterally administered, a single dose of the compound or its salt may vary depending on subject of administration, target disease, etc. When the compound or its salt that regulates (e.g., promotes) the activity of the protein of the present invention is administered in the form of an injection to adult (as 60 kg) for the purpose of treatment of Alzheimer's disease, it is advantageous to administer the compound or its salt by intravenous injection generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

### (3) Quantification of the protein of the present invention, its partial peptide, or its salt

The antibody to the protein of the present invention (sometimes simply referred to as the antibody of the present invention) is capable of specifically recognizing the protein of the present invention and can thus be used for quantification of the protein of the present invention in a test sample fluid, particularly for quantification by the sandwich immunoassay.

That is, the present invention provides:
(i) a method for quantification of the protein of the present invention in a test sample fluid, which comprises competitively reacting the antibody of the present invention, a test sample fluid and a labeled form of the protein of the present invention, and measuring the ratio of the labeled the protein of the present invention bound to said antibody; and
(ii) a method for quantification of the protein of the present invention in a test sample fluid, which comprises reacting the test sample fluid simultaneously or continuously with the antibody of the present invention immobilized on a carrier and a labeled form of the antibody of the present invention, and then measuring the activity of the labeling agent on the insoluble carrier.

In the quantification method in the above-mentioned (ii), it is desirable that one antibody is an antibody recognizing the N-terminal region of the protein of the present invention, and the other antibody is an antibody reacting with the C-terminal region of the protein of the present invention.

The monoclonal antibody to the protein of the present invention (hereinafter sometimes referred to as the monoclonal antibody of the present invention) may be used to quantify the protein of the present invention. Besides, the protein of the present invention may also be detected by means of tissue staining. For these purposes, the antibody molecule per se may be used or F(ab')₂, Fab' or Fab fractions of the antibody molecule may be used as well.

There is no particular limitation to the method of quantifying the protein using the antibody of the present invention; any method may be used so far as it relates to a method in which the amount of antibody, antigen or antibody-antigen complex can be detected by a chemical or a physical means, depending on or corresponding to the amount of antigen (e.g., the amount of the protein) in a test sample fluid to be assayed, and then calculated using a standard curve prepared by a standard solution containing the known amount of antigen. Advantageously used are, for example, nephrometry, competitive method, immunometric method and sandwich method; in terms of sensitivity and specificity, the sandwich method which will be later described is particularly preferred.

Examples of the labeling agent used in the assay method using the labeling substance are radioisotopes (for example, [¹²⁵I], [¹³¹I], [³H], [¹⁴C], [³²P], [³³P], [³⁵S] , etc.), fluorescent substances [for example, cyanine fluorescent dyes (e.g., Cy2, Cy3, Cy5, Cy5.5, Cy7 (Amersham Bioscience) etc), fluorescamine, fluorescein isothiocyanate etc.], enzymes (for example, β-galactosidase, β-glucosidase, alkaline phosphatase, peroxidase, malate dehydrogenase etc), luminescent substances (for example, luminol, a luminol derivative, luciferin, lucigenin etc.), biotin, and lanthanide elements. Furthermore, the biotin-avidin system may also be used for binding of an antibody or antigen to a labeling agent.

In the immobilization of antigens or antibodies, physical adsorption may be used. Alternatively, chemical binding conventionally used for immobilization of the protein or enzymes may be used as well. Examples of the carrier include insoluble polysaccharides such as agarose, dextran and cellulose; synthetic resins such as polystyrene, polyacrylamide, silicone, etc.; glass; and the like.

In the sandwich method, a test sample fluid is reacted with an immobilized form of the monoclonal antibody of the present invention (primary reaction), then reacted with another labeled form of the monoclonal antibody of the present invention (secondary reaction) and the activity of the labeling agent on the insoluble carrier is assayed, whereby the amount of the protein of the present invention in the test sample fluid can be quantified. The primary and secondary reactions may be carried out in a reversed order, simultaneously or sequentially with an interval. The type of the labeling agent and the method for immobilization may be effected by modifications of those described hereinabove. In the immunoassay by the sandwich method, it is not always necessary that the antibody used for the labeled antibody and for the solid phase should be one type or one species, and a mixture of two or more antibodies may be used as well for the purpose of improving the measurement sensitivity, etc.

In the method for assaying the protein of the present invention by the sandwich method according to the present invention, preferred monoclonal antibodies of the present invention used for the primary and the secondary reactions are antibodies whose binding sites to the protein are different from one another. Thus, the antibodies used in the primary and the secondary reactions are those wherein when the antibody used in the secondary reaction recognizes the C-terminal region of the protein of the present invention, the antibody recognizing the site other than the C-terminal region, e.g., recognizing the N-terminal region, is preferably used in the primary reaction.

The monoclonal antibody of the present invention may be used in an assay system other than the sandwich method, such as a competitive method, an immunometric method, nephrometry, etc.

In the competitive method, an antigen in a test sample fluid and a labeled antigen are competitively reacted with an antibody, then the unreacted labeled antigen (F) and the labeled antigen bound to the antibody (B) are separated (i.e., B/F separation) and the labeled amount of either B or F is measured to determine the amount of the antigen in the test sample fluid. In the reactions for such a method, there are a liquid phase method in which a soluble antibody is used as the antibody and the B/F separation is effected by polyethylene glycol while a second antibody to the antibody described above is used, and a solid phase method in which an immobilized antibody is used as the first antibody, or a soluble antibody is used as the first antibody while an immobilized antibody is used as the second antibody.

In the immunometric method, an antigen in a test sample fluid and an immobilized antigen are competitively reacted with a given amount of a labeled antibody followed by separating the solid phase from the liquid phase; or an antigen in a test sample fluid and an excess amount of labeled antibody are reacted, then an immobilized antigen is added to bind an unreacted labeled antibody to the solid phase, and the solid phase is separated from the liquid phase. Thereafter, the labeled amount of any of the phases is measured to determine the antigen amount in the test sample fluid.

In the nephrometry, the amount of insoluble sediment, which is produced as a result of the antigen-antibody reaction in a gel or in a solution, is measured. Even when the amount of an antigen in a test sample fluid is small and only a small amount of the sediment is obtained, laser nephrometry utilizing laser scattering can be suitably used.

In applying each of those immunoassays to the quantification method of the present invention, any special conditions or operations are not required to set forth. The assay system for the protein of the present invention may be constructed in addition to conditions or operations conventionally used for each of the methods, taking the technical consideration by one skilled in the art into account. For the details of such conventional technical means, a variety of reviews, reference books, etc. may be referred to.

For example, reference can be made to Hiroshi Irie (ed.): "Radioimmunoassay" (published by Kodansha, 1974); Hiroshi Irie (ed.): "Radioimmunoassay; Second Series" (published by Kodansha, 1979); Eiji Ishikawa, et al. (ed.): "Enzyme Immunoassay" (published by Igaku Shoin, 1978); Eiji Ishikawa, et al. (ed.): "Enzyme Immunoassay" (Second Edition) (published by Igaku Shoin, 1982); Eiji Ishikawa, et al. (ed.): "Enzyme Immunoassay" (Third Edition) (published by Igaku Shoin, 1987); "Methods in Enzymology" Vol. 70 (Immuochemical Techniques (Part A)); ibid., Vol. 73 (Immunochemical Techniques (Part B)); ibid., Vol. 74 (Immunochemical Techniques (Part C)); ibid., Vol. 84 (Immunochemical Techniques (Part D: Selected Immunoassays)); ibid., Vol. 92 (Immunochemical Techniques (Part E: Monoclonal Antibodies and General Immunoassay Methods)); ibid., Vol. 121 (Immunochemical Techniques (Part I: Hybridoma Technology and Monoclonal Antibodies)) (published by Academic Press); etc.

As described above, the protein of the present invention can be quantified with high sensitivity, using the antibody of the present invention.

Furthermore, when an increase or decrease in the concentration of the protein of the present invention is detected by quantifying the concentration of the protein of the present invention using the antibody of the present invention, it can be diagnosed that there occur or will highly likely occur neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

The antibodies of the present invention can also be used for specifically detecting the protein of the present invention present in test samples such as body fluids or tissues. The antibodies may also be used for preparation of antibody columns for purification of the protein of the present invention, for detection of the protein of the present invention in each fraction upon purification, and for analysis of the behavior of the protein of the present invention in the test cells.

### (4) Gene diagnostic agent

By using the DNA of the present invention as a probe, an abnormality (gene abnormality) of the DNA or mRNA encoding the protein of the present invention or its partial peptide in human or warm-blooded animals (e.g., rat, mouse, guinea pig, rabbit, chicken, sheep, swine, bovine, horse, cat, dog, monkey, chimpanzee etc.) can be detected. Therefore, the DNA of the present invention is useful as a gene diagnostic agent for detecting damages to the DNA or mRNA, its mutation, or decreased expression, increased expression, overexpression, etc. of the DNA or mRNA, and so on.

The gene diagnosis described above using the DNA of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)), etc.

When overexpression or decreased expression of the protein is detected, e.g., by the Northern hybridization or when DNA mutation is detected by the PCR-SSCP assay, it can be diagnosed that it is highly likely to suffer from neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

### (5) Pharmaceutical comprising the antisense polynucleotide

The antisense polynucleotide of the present invention that binds complementarily to the DNA of the present invention to inhibit expression of the DNA is low-toxic and can suppress the functions of the protein of the present invention or the DNA of the present invention in the body, and can thus be used as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), or as an cell-death inhibitor.

When the antisense polynucleotide is used as the aforesaid prophylactic/therapeutic agent, it can be formed into a pharmaceutical preparation and administered in publicly known methods.

For example, the antisense polynucleotide itself, or the antisense polynucleotide inserted into an appropriate vector such as retrovirus vector, adenovirus vector, adenovirus-associated virus vector, etc., is administered orally or parenterally to human or other warm-blooded animal (e.g., rat, rabbit, sheep, swine, bovine, cat, dog, monkey, etc.) in a conventional manner. The antisense polynucleotide may also be administered as it is, or prepared into pharmaceutical preparations together with physiologically acceptable carriers such as adjuvants to assist its uptake, and such preparations are administered by gene gun or through a catheter like a hydrogel catheter. Alternatively, the antisense polynucleotide may be prepared in the form of aerosol and intratracheally administered as an inhalant.

For the purposes of improvement of movement in the body, prolongation of the half-life, and improvement of incorporation into cells, the antisense polynucleotide may be administered such as intravenously, subcutaneously or into affected lesions directly or after formation into pharmaceutical preparations (injections) together with carriers such as liposomes.

The dose of the antisense polynucleotide may vary depending on target disease, subject of administration, route for administration, etc. When the antisense polynucleotide of the present invention is administered for the purpose of treatment of Alzheimer's disease, the antisense polynucleotide is administered to adult (60 kg body weight) usually in a daily dose of approximately 0.1 to 100 mg.

In addition, the antisense polynucleotide may also be employed as an oligonucleotide probe for diagnosis to examine the presence of the DNA of the present invention in tissues or cells, or the states of its expression.

Double-stranded RNA comprising a part of RNA encoding the protein of the present invention or a ribozyme comprising a part of RNA encoding the protein of the present invention, similar to the antisense polynucleotide described above, can suppress the expression of the gene for the present invention and can, in vivo, suppress the functions of the protein of the present invention or the DNA of the present invention, and can thus be used as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

According to known methods (for example, Nature, 411, 494, 2001), the double-stranded RNA can be produced by designing it on the basis of the sequence of the polynucleotide of the present invention.

According to known methods (for example, TRENDS in Molecular Medicine, 7, 221, 2001), the ribozyme can be produced by designing it on the basis of the sequence of the polynucleotide of the present invention. For example, the ribozyme can be produced by partially replacing a known ribozyme sequence by a part of RNA encoding the protein of the present invention. The part of RNA encoding the protein of the present invention includes a sequence (RNA fragment) adjacent to the cleavage site on the RNA of the present invention which can be cleaved with a known ribozyme.

When the double-stranded RNA or the ribozyme is to be used as the aforesaid prophylactic/therapeutic agent, it can be formed into a pharmaceutical preparation and administered in the same manner as for the antisense polynucleotide.

### (6) Pharmaceutical preparation comprising the antibody of the present invention

The antibody of the present invention having an action of neutralizing the activity of the protein of the present invention is useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), an cell-death inhibitor, etc.

The antibody of the present invention may be administered in itself or as an appropriate pharmaceutical composition. The pharmaceutical composition used for the administration described above comprises the antibody or its salt and a pharmacologically acceptable carrier, a diluent or excipient. Such a composition is provided in the preparation suitable for oral or parenteral administration.

Examples of the composition for oral administration include solid or liquid preparations, specifically tablets (including dragees and film-coated tablets), pills, granules, powdery preparations, capsules (including soft capsules), syrup, emulsions, suspensions, etc. Such a composition is manufactured by publicly known methods and comprises a vehicle, a diluent or an excipient conventionally used in the field of pharmaceutical preparations. Examples of the vehicle or excipient for tablets are lactose, starch, sucrose, magnesium stearate, etc.

Examples of the composition for parenteral administration that can be used are injections, suppositories, etc. and the injections include the form of intravenous, subcutaneous, transcutaneous, intramuscular and drip injections. Such injections are prepared by publicly known methods, e.g., by dissolving, suspending or emulsifying the aforesaid antibody or its salts in a sterile aqueous or oily liquid medium used usually in injections. For the aqueous medium for injection, for example, physiological saline and isotonic solutions containing glucose and other adjuvant, etc. are used. Appropriate dissolution aids, for example, an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. may be used in combination. For the oily solution, for example, sesame oil, soybean oil, etc. are used, and dissolution aids such as benzyl benzoate, benzyl alcohol, etc. may be used in combination. The thus prepared liquid for injection is normally filled in an appropriate ampoule. The suppository used for rectal administration is prepared by mixing the aforesaid antibody or its salts with conventional suppository base.

The oral or parenteral pharmaceutical composition described above is advantageously prepared in a unit dosage form suitable for the dose of the active ingredient. Examples of such unit dosage form include tablets, pills, capsules, injections (ampoules), suppositories, etc. It is preferred that the antibody described above is contained generally in a dose of approximately 5 to 500 mg per unit dosage form, approximately 5 to 100 mg especially for injections and approximately 10 to 250 mg for other preparations.

Each composition described above may further comprise other active components unless formulation with the antibody causes any adverse interaction.

The prophylactic/therapeutic agent comprising the antibody of the present invention is low toxic, and can be administered orally or parenterally (for example through intravenous administration) as a liquid preparation directly or as a pharmaceutical composition in a suitable form, into humans or mammals (e.g., rat, rabbit, sheep, swine, bovine, cat, dog, monkey, etc.). The dose of the antibody of the present invention may vary depending on subject of administration, target disease, symptom, route for administration, etc. When the antibody of the present invention is administered for the purpose of treatment of Alzheimer's disease in adult, it is advantageous to administer the antibody of the present invention as an injection once to about 5 times per day, preferably once to 3 times per day, in a daily dose of approximately 0.01 to 20 mg/kg, preferably approximately 0.1 to 10 mg/kg, more preferably approximately 0.1 to 5 mg/kg. In other parenteral administration or oral administration, the corresponding dose can be administered. When the symptom is particularly severe, the dose may be increased depending on the symptom.

The antibody of the present invention is also useful as a diagnostic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), etc.

### (7) Animal bearing the DNA of the present invention

The present invention provides a non-human mammal bearing the DNA encoding the protein of the present invention, which is exogenous (hereinafter simply referred to as the exogenous DNA of the present invention) or its mutant DNA (sometimes simply referred to as the exogenous mutant DNA of the present invention).

Thus, the present invention provides:
(1) a non-human mammal having the exogenous DNA of the present invention or its mutant DNA;
(2) the mammal according to (1), wherein the non-human mammal is a rodent;
(3) the mammal according to (2), wherein the rodent is a mouse or rat; and
(4) a recombinant vector comprising the exogenous DNA of the present invention or its mutant DNA and capable of expression in a mammal.

The non-human mammal having the exogenous DNA of the present invention or its mutant DNA (hereinafter simply referred to as the DNA transgenic animal of the present invention) can be created by transfecting the desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase) by standard means such as the calcium phosphate method, the electric pulse method, the lipofection method, the agglutination method, the microinjection method, the particle gun method, the DEAE-dextran method, etc. Also, it is possible to transfect the exogenous DNA of the present invention into a somatic cell, a living organ, a tissue cell or the like, by the DNA transfection methods, and utilize the transformant for cell culture, tissue culture, etc. In addition, these cells may be fused with the above-described germinal cell by a publicly known cell fusion method to create the transgenic animal of the present invention.

Examples of the non-human mammal that can be used include bovine, swine, sheep, goat, rabbits, dogs, cats, guinea pigs, hamsters, mice, rats and the like. Above all, preferred are rodents, especially mice (e.g., C57BL/6 strain, DBA2 strain, etc. for a pure line and for a cross line, B6C3F₁ strain, BDF₁ strain, B6D2F₁ strain, BALB/c strain, ICR strain, etc.) or rats (Wistar, SD, etc.) and the like, since they are relatively short in ontogeny and life cycle from a standpoint of creating model disease animals, and are easy in breeding.

"Mammals" in a recombinant vector that can be expressed in mammals include human etc. in addition to the aforesaid non-human mammals.

The exogenous DNA of the present invention refers to the DNA of the present invention that is once isolated and extracted from mammals, not the DNA of the present invention inherently possessed by the non-human mammals.

The mutant DNA of the present invention includes mutants resulting from variation (e.g., mutation, etc.) in the nucleotide sequence of the original DNA of the present invention, specifically DNAs resulting from base addition, deletion, substitution with other bases, etc. and further including abnormal DNA.

The abnormal DNA is intended to mean the DNA that expresses abnormal protein of the present invention and exemplified by such a DNA that expresses the protein suppressing the functions of the normal protein of the present invention, or the like.

The exogenous DNA of the present invention may be any one of those derived from a mammal of the same species as, or a different species from, the mammal as the target animal. In transfecting the DNA of the present invention to the target animal, it is generally advantageous to use the DNA as a DNA construct in which the DNA is ligated downstream from a promoter capable of expressing the DNA in the target animal. For example, in the case of transfecting the human DNA of the present invention, a DNA transgenic mammal that expresses the DNA of the present invention to a high level can be prepared by microinjecting a DNA construct (e.g., vector, etc.) ligated with the human DNA of the present invention into a fertilized egg of the target mammal, e.g., a fertilized egg of mouse, downstream from the various promoters capable of expressing the DNA derived from various mammals (e.g., rabbits, dogs, cats, guinea pigs, hamsters, rats, mice, etc.) having the DNA of the present invention highly homologous to the human DNA.

As expression vectors for the protein of the present invention, there are Escherichia coli-derived plasmids, Bacillus subtilis-derived plasmids, yeast-derived plasmids, bacteriophages such as λ phage, etc., retroviruses such as Moloney leukemia virus, etc., animal viruses such as vaccinia virus, baculovirus, etc. Of these vectors, Escherichia coli-derived plasmids, Bacillus subtilis-derived plasmids, yeast-derived plasmids, etc. are preferably used.

Examples of these promoters for regulating the DNA expression include (i) promoters for DNA derived from viruses (e.g., simian virus, cytomegalovirus, Moloney leukemia virus, JC virus, breast cancer virus, poliovirus, etc.), and (ii) promoters derived from various mammals (humans, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na, K-ATPase), neurofilament light chain, metallothioneins I and IIA, metalloproteinase 1 tissue inhibitor, MHC class I antigen (H-2L), H-ras, renin, dopamine β-hydroxylase, thyroid peroxidase (TPO), polypeptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Preferable among these are cytomegalovirus promoters, human peptide elongation factor 1α (EF-1α) promoters, human and chicken β actin promoters etc., which can achieve high expression in the whole body.

It is preferred that the vectors described above have a sequence for terminating the transcription of the desired messenger RNA in the DNA transgenic animal (generally called a terminator), for example, a sequence of each DNA derived from viruses and various mammals. SV40 terminator of the simian virus, etc. are preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purposes.

The normal translational region of the protein of the present invention can be prepared as the whole or a part of genomic DNA from DNA derived from liver, kidney, thyroid cells, fibroblasts etc. derived from humans or mammals (for example, rabbit, dog, cat, guinea pig, hamster, rat, mouse etc.) and a wide variety of commercial DNA libraries, or from complementary DNA as a starting material prepared by a known method from RNA derived from liver, kidney, thyroid cells, fibroblasts etc. As the extraneous abnormal DNA, a translational region can be prepared by point mutation of the normal translational region of the protein obtained from the above cells or tissues.

The translational region can be prepared, as a DNA construct capable of being expressed in the transgenic animal, by a conventional DNA engineering technique, in which the DNA is ligated downstream from the aforesaid promoter and if desired, upstream from the translation termination site.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal, in which the normal exogenous DNA of the present invention has been transfected, can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells of the target mammal. The fact that the exogenous DNA of the present invention is excessively present in the germinal cells of the prepared animal after transfection means that all of the offspring of the animal prepared have the exogenous DNA of the present invention excessively in all of the germinal cells and somatic cells thereof. The offspring of the animal of this kind that inherits the exogenous DNA of the present invention excessively have the DNA of the present invention in all of the germinal cells and somatic cells thereof.

By obtaining a homozygotic animal having the transfected DNA in both of homologous chromosomes and mating a male and female of the animal, all offspring can be passaged to excessively retain the DNA.

In a non-human mammal bearing the normal DNA of the present invention, the normal DNA of the present invention is expressed to a high level, and may eventually develop the hyperfunction of the protein of the present invention by promoting the functions of endogenous normal DNA. Therefore, the animal can be utilized as a pathologic model animal for such a disease. Specifically, using the normal DNA transgenic animal of the present invention, it becomes possible to elucidate the hyperfunction of the protein of the present invention and to clarify the pathological mechanism of the disease associated with the protein of the present invention and to determine how to treat these diseases.

Furthermore, since a mammal transfected with the exogenous normal DNA of the present invention exhibits an increasing symptom of the protein of the present invention, the animal is usable for screening of prophylactic/therapeutic agents for the disease associated with the protein of the present invention, for example, neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

On the other hand, non-human mammal having the exogenous abnormal DNA of the present invention can be passaged under normal breeding conditions as the DNA-bearing animal by confirming the stable retaining of the exogenous DNA via crossing. In addition, the objective exogenous DNA can be utilized as a starting material by inserting the objective exogenous DNA into the plasmid described above. The DNA construct with a promoter can be prepared using conventional DNA engineering techniques. The transfection of the abnormal DNA of the present invention at the fertilized egg cell stage is preserved to be present in all of the germinal and somatic cells of the mammals to be targeted. The fact that the abnormal DNA of the present invention is present in the germinal cells of the animal after DNA transfection means that all of the offspring of the prepared animal have the abnormal DNA of the present invention in all of the germinal and somatic cells. The offspring of such an animal that inherits the exogenous DNA of the present invention has the abnormal DNA of the present invention in all the germinal and somatic cells. A homozygous animal having the introduced DNA on both of homologous chromosomes can be acquired and then by mating these male and female animals, all the offspring can be bred to have the DNA.

Since the non-human mammal having the abnormal DNA of the present invention expresses the abnormal DNA of the present invention at a high level, the animal may cause the function inactive type inadaptability of the protein of the present invention by inhibiting the functions of the endogenous normal DNA, and can be utilized as its disease model animal. For example, using the abnormal DNA-transferred animal of the present invention, it is possible to elucidate the mechanism of the function inactive type inadaptability of the protein of the present invention and to study a method for treatment of this disease.

In its specific applicability, the transgenic animal of the present invention expressing the abnormal DNA of the present invention to a high level is also expected to serve as a model for the elucidation of the mechanism of the functional inhibition (dominant negative effect) of the normal protein of the present invention by the abnormal protein of the present invention in the function inactive type inadaptability of the protein of the present invention.

A mammal bearing the abnormal exogenous DNA of the present invention has a symptom of increasing the protein of the present invention in a free form, and is thus utilizable in a test of screening prophylactic/therapeutic agents for the function inactive type inadaptability of the protein of the present invention, for example prophylactic/therapeutic agents for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

Other potential applications of two kinds of the DNA transgenic animals of the present invention described above further include:
(i) Use as a cell source for tissue culture,
(ii) Elucidation of the relation to the protein that is specifically expressed or activated by the protein of the present invention, by direct analysis of DNA or RNA in tissues of the DNA transgenic animal of the present invention or by analysis of the peptide tissues expressed by the DNA,
(iii) Research on the function of cells derived from tissues that are usually cultured only with difficulty, using cells in tissues bearing the DNA cultured by a standard tissue culture technique,
(iv) Screening a drug that enhances the functions of cells using the cells described in (iii) above, and
(v) Isolation and purification of the mutant protein of the present invention and preparation of an antibody thereto.

Furthermore, clinical conditions of a disease associated with the protein of the present invention, including the function inactive type inadaptability to the protein of the present invention, can be examined by using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading it with a proteinase such as trypsin, etc., followed by establishing the line of culturing or cultured cells. Furthermore, the DNA transgenic animal of the present invention can serve to identify cells capable of producing the protein of the present invention, and to study in association with apoptosis, differentiation or propagation or on the mechanism of signal transduction in these properties to inspect any abnormality therein. Thus, the DNA transgenic animal can provide an effective research material for the protein of the present invention and for investigation of the function and effect thereof.

To develop a drug for the treatment of diseases associated with the protein of the present invention, including the function inactive type inadaptability to the protein of the present invention, using the DNA transgenic animal of the present invention, an effective and rapid method for screening a drug for the treatment of the diseases can be provided by using the method for inspection, the method for quantification etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### (8) Knockout animal

The present invention provides a non-human mammal embryonic stem cell bearing the DNA of the present invention inactivated and a non-human mammal deficient in expressing the DNA of the present invention.

Thus, the present invention provides:
(1) a non-human mammal embryonic stem cell in which the DNA of the present invention is inactivated;
(2) the embryonic stem cell according to (1), wherein the DNA is inactivated by introducing a reporter gene (e.g., β-galactosidase gene derived from Escherichia coli);
(3) the embryonic stem cell according to (1), which is resistant to neomycin;
(4) the embryonic stem cell according to (1), wherein the non-human mammal is a rodent;
(5) an embryonic stem cell according to (4), wherein the rodent is a mouse;
(6) a non-human mammal deficient in expressing the DNA of the present invention,
   wherein the DNA of the present invention is inactivated;
(7) the non-human mammal according to (6), wherein the DNA is inactivated by inserting a reporter gene (e.g., β-galactosidase derived from Escherichia coli) therein and the reporter gene is capable of being expressed under the control of a promoter for the DNA of the present invention;
(8) the non-human mammal according to (6), which is a rodent;
(9) the non-human mammal according to (8), wherein the rodent is a mouse; and
(10) a method for screening a compound or its salt that promotes or inhibits the promoter activity for the DNA of the present invention, which comprises administering a test compound to the animal of (7) and detecting expression of the reporter gene.

The non-human mammalian embryonic stem cell, in which the DNA of the present invention is inactivated, refers to a non-human mammalian embryonic stem cell that suppresses the ability of the non-human mammalian to express the DNA by artificially mutating the DNA of the present invention possessed in the non-human mammal, or the DNA has no substantial ability to express the protein of the present invention (hereinafter sometimes referred to as the knockout DNA of the present invention) by substantially inactivating the activities of the protein of the present invention encoded by the DNA (hereinafter merely referred to as ES cell).

As the non-human mammalian, the same examples as described above apply. Techniques for artificially mutating the DNA of the present invention include deletion of a part or all of the DNA sequence and insertion of, or substitution with, other DNA, e.g., by genetic engineering. By these mutations, the knockout DNA of the present invention may be prepared, for example, by shifting the reading frame of a codon or by destroying the function of a promoter or exon.

Specifically, the non-human mammalian embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention possessed by the target non-human mammal, inserting a DNA strand (hereinafter simply referred to as targeting vector) having a DNA sequence constructed so as to eventually destroy the gene by inserting into its exon site a chemical resistant gene such as a neomycin resistant gene or a hygromycin resistant gene, or a reporter gene such as lacZ (β-galactosidase gene) or cat (chloramphenicol acetyltransferase gene), etc. thereby destroying the functions of exon, or by inserting into the intron site between exons a DNA sequence which terminates gene transcription (e.g., polyA-added signal, etc.) thereby disabling the synthesis of complete messenger RNA, into a chromosome of the animal cells by, e.g., homologous recombination. The thus obtained ES cells are analyzed by the Southern hybridization using as a probe a DNA sequence on or near the DNA of the present invention, or by PCR using as primers a DNA sequence on the targeting vector and another DNA sequence near the DNA of the present invention which is not included in the targeting vector, and the knockout ES cell of the present invention is selected.

The parent ES cells to inactivate the DNA of the present invention by homologous recombination, etc. may be of a strain already established as described above, or may be originally established in accordance with a modification of the known method by Evans and Kaufman. For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum collection per C57BL/6 mouse or C57BL/6 has been improved by crossing with DBA/2, may be preferably used, instead of obtaining a pure line of ES cells with the clear immunological genetic background. The BDF₁ mouse is advantageous in that when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. A large number of early stage embryos may be acquired more efficiently, by collecting the embryos of the 8-cell stage and using the same after culturing until the blastocyte stage.

Although the ES cells used may be of either sex, male ES cells are generally more convenient for generation of a germ cell line chimera and are therefore preferred. It is desirable to identify sexes as soon as possible also in order to save painstaking culture time.

As an example of the method for sex identification of the ES cell, mention may be made of a method in which a gene in the sex-determining region on the Y-chromosome is amplified by PCR and detected. When this method is used, ES cells (about 50 cells) corresponding to almost 1 colony are sufficient, whereas karyotype analysis hitherto required about 10⁶ cells; therefore, the first selection of ES cells at the early stage of culture can be based on sex identification, and male cells can be selected early, which saves a significant amount of time at the early stage of culture.

Second selection can be achieved by, for example, chromosome number confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and about 90% air) in the presence of LIF (1-10000 U/ml) on appropriate feeder cells such as STO fibroblasts, treated with a trypsin/EDTA solution (normally about 0.001 to about 0.5% trypsin/about 0.1 to 5 mM EDTA, preferably about 0.1% trypsin/about 1 mM EDTA) at the time of passage to obtain separate single cells, which are then seeded on freshly prepared feeder cells. This passage is normally conducted every 1 to 3 days; it is desirable that cells be observed at passage and cells found to be morphologically abnormal in culture, if any, be abandoned.

By allowing ES cells to reach a high density in mono-layers or to form cell aggregates in suspension under appropriate conditions, it is possible to differentiate them to various cell types, for example, parietal and visceral muscles, cardiac muscle or the like [Nature, 292, 154, 1981; Proc. Natl. Acad. Sci. U.S.A., 78, 7634, 1981; Journal of Embryology Experimental Morphology, 87, 27, 1985]. The cells deficient in expression of the DNA of the present invention, which are obtainable from the differentiated ES cells of the present invention, are useful for studying the protein of the present invention in vitro cytologically or molecular biologically.

The non-human mammal deficient in expression of the DNA of the present invention can be identified from a normal animal by measuring the amount of mRNA in the subject animal by a publicly known method, and indirectly comparing the levels of expression.

As the non-human mammal, the same examples supra apply.

With respect to the non-human mammal deficient in expression of the DNA of the present invention, the DNA of the present invention can be made knockout by transfecting a targeting vector, prepared as described above, to mouse embryonic stem cells or mouse oocytes thereof, and conducting homologous recombination in which a targeting vector DNA sequence, wherein the DNA of the present invention is inactivated by the transfection, is replaced with the DNA of the present invention on a chromosome of a mouse embryonic stem cell or mouse oocyte.

The cells with the DNA of the present invention in which the DNA of the present invention is rendered knockout can be identified by the Southern hybridization analysis using as a probe a DNA sequence on or near the DNA of the present invention, or by PCR analysis using as primers a DNA sequence on the targeting vector and another DNA sequence which is not included in the DNA of the present invention derived from mouse, which is used as the targeting vector. When non-human mammalian embryonic stem cells are used, the cell line wherein the DNA of the present invention is inactivated is cloned by homologous recombination; the resulting cloned cell line is injected to, e.g., a non-human mammalian embryo or blastocyte, at an appropriate stage such as the 8-cell stage. The resulting chimeric embryos are transplanted to the uterus of the pseudo-pregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual in which all tissues are composed of cells having an artificially mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between the heterozygotes.

When an oocyte is used, a DNA solution may be injected, e.g., to the prenucleus by microinjection thereby obtaining a transgenic non-human mammal having a targeting vector introduced into its chromosome. From such transgenic non-human mammals, those having a mutation at the locus of the DNA of the present invention can be obtained by selection based on homologous recombination.

Individuals wherein the DNA of the present invention is rendered knockout permit passage rearing under ordinary rearing conditions, after it is confirmed that in the animal individuals obtained by their crossing, the DNA has been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammalian embryonic stem cell, in which the DNA of the present invention is inactivated, is very useful for preparing a non-human mammal deficient in expression of the DNA of the present invention.

Since the non-human mammal, in which the DNA of the present invention fails to express, lacks various biological activities induced by the protein of the present invention, such an animal can be a disease model suspected of inactivated biological activities of the protein of the present invention and thus, offers an effective study to investigate causes for and therapy for these diseases.

### (8a) Method for screening of compounds having therapeutic/prophylactic effects for diseases caused by deficiency, damages, etc. of the DNA of the present invention

The non-human mammal deficient in expression of the DNA of the present invention can be used to screen compounds having therapeutic/prophylactic effects for diseases caused by deficiency, damages, etc. of the DNA of the present invention.

That is, the present invention provides a method for screening of a compound or its salt having therapeutic/prophylactic effects for diseases caused by deficiency, damages, etc. of the DNA of the present invention, for example neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.), which comprises administering a test compound to the non-human mammal deficient in expression of the DNA of the present invention, and observing and measuring a change having occurred in the animal.

As the non-human mammal deficient in expression of the DNA of the present invention used for the screening method, the same examples as given hereinabove apply.

Examples of the test compounds include peptides, proteins, non-peptide compounds, synthetic compounds, fermentation products, cell extracts, plant extracts, animal tissue extracts, blood plasma, etc. and these compounds may be novel compounds or publicly known compounds.

Specifically, the non-human mammal deficient in the expression of the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be tested with a test compound, for example, oral administration, intravenous injection, etc. are applied and the treatment is appropriately selected depending upon conditions of the test animal, properties of the test compound, etc. Furthermore, the amount of a test compound administered can be appropriately selected depending on administration route, nature of the test compound, or the like.

For example, when a compound having a prophylactic/therapeutic effect on neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.) is screened, a test compound is administered into a non-human mammalian animal deficient in expressing DNA encoding the protein of the present invention, and then a difference in the number of dead nerve cells from the group not given the test compound and a difference in mass of various proteins is observed with time in the tissues.

In the screening method, when the disease symptom of a test animal is ameliorated by about 10% or more, preferably about 30% or more, more preferably about 50% or more by administering a test compound to the test animal, the test compound can be selected as a compound that has therapeutic/prophylactic effects on the disease.

The compound obtained by the screening method is a compound selected from the test compounds, and can be used for example as a low toxic and safe pharmaceutical preparation such as a prophylactic/therapeutic agent for diseases caused by deficiency in or damage to the protein of the present invention. Further, a compound derived from the compound obtained by the screening can also be similarly used.

The compound obtained by the screening method may form a salt, and as the salts of the compound, there may be used salts with physiologically acceptable acids (e.g., inorganic acids or organic acids) or bases (e.g., alkali metals), preferably physiologically acceptable acid addition salts. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

A pharmaceutical preparation comprising the compound or salts thereof obtained by the above screening method may be manufactured in a manner similar to the above-described method for preparing the pharmaceutical preparation comprising the protein of the present invention.

Since the pharmaceutical preparation thus obtained is safe and low toxic, it can be administered to humans or other mammals (e.g., rat, mouse, guinea pig, rabbit, sheep, swine, bovine, horse, cat, dog, monkey, etc.).

The dose of the compound or its salt may vary depending on target disease, subject of administration, route for administration, etc. When the compound is orally administered, the compound is administered to a patient (as 60 kg) with Alzheimer's disease generally in a daily dose of approximately 0.1 to 100 mg, preferably approximately 1.0 mg to 50 mg, more preferably approximately 1.0 to 20 mg. When the compound is parenterally administered, a single dose of the compound may vary depending on subject of administration, target disease, etc. When the compound is administered in the form of an injection to a patient (as 60 kg) with Alzheimer's disease, it is advantageous to administer the compound by intravenous injection generally in a daily dose of approximately 0.01 to 30 mg, preferably approximately 0.1 to 20 mg, more preferably approximately 0.1 to 10 mg. For other animal species, the corresponding dose as converted per 60 kg weight can be administered.

### (8b) Method of screening a compound that promotes or inhibits the activities of a promoter for the DNA of the present invention

The present invention provides a method of screening a compound or its salt that promotes or inhibits the activities of a promoter for the DNA of the present invention, which comprises administering a test compound to a non-human mammal deficient in expression of the DNA of the present invention and detecting expression of the reporter gene.

In the screening method described above, the non-human mammal deficient in expression of the DNA of the present invention is selected from the aforesaid non-human mammals deficient in expression of the DNA of the present invention in which the DNA of the present invention is inactivated by introducing a reporter gene and the reporter gene can be expressed under the control of a promoter for the DNA of the present invention.

The same examples given above for the test compound apply to the test compound.

As the reporter gene, the same specific examples given above apply to the reporter gene, with β-galactosidase (lacZ), soluble alkaline phosphatase gene, luciferase gene, etc. being preferred.

In the non-human mammal deficient in expression of the DNA of the present invention wherein the DNA of the present invention is replaced by a reporter gene, the reporter gene is present under the control of a promoter for the DNA of the present invention. Thus, the activity of the promoter can be detected by tracing the expression of a substance encoded by the reporter gene.

For example, when a part of the DNA region encoding the protein of the present invention is replaced by, e.g., β-galactosidase gene (lacZ) derived from Escherichia coli, β-galactosidase is expressed in a tissue where the protein of the present invention should originally be expressed, in place of the protein of the present invention. Thus, the expression state of the protein of the present invention can be readily observed in vivo in an animal, by staining with a reagent, e.g., 5-bromo-4-chloro-3-indolyl-β-galactopyranoside (X-gal), which is a substrate for β-galactosidase. Specifically, a mouse deficient in the protein of the present invention, or its tissue section, is fixed with glutaraldehyde, etc. After washing with phosphate buffered saline (PBS), the system is reacted with a staining solution containing X-gal at room temperature or about 37°C for approximately 30 minutes to 1 hour. After the β-galactosidase reaction is terminated by washing the tissue preparation with 1 mM EDTA/PBS solution, the color formed is observed. Alternatively, mRNA encoding lacZ may be detected in a conventional manner.

The compound or salts thereof obtained using the screening methods supra are compounds selected from the test compounds described above, which promote or inhibit the promoter activity for the DNA of the present invention.

The compounds obtained by the screening methods may form salts, and as the salts of the compounds, use is made of salts with physiologically acceptable acids (e.g., inorganic acids) or bases (e.g., alkali metals), and physiologically acceptable acid addition salts are preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The compound or its salt that promotes or inhibits the promoter activity for the DNA of the present invention can regulate the expression of the protein of the present invention and regulate the functions of the protein of the present invention, and is thus useful as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.).

Further, a compound derived from the compound obtained in the above screening can also be used similarly.

The pharmaceutical preparation comprising the compound or its salt obtained by the screening method can be produced in a manner similar to the method for preparing the pharmaceutical preparation comprising the protein of the present invention or its salt.

The thus obtained pharmaceutical preparation is safe and low toxic, and can thus be administered to, for example, humans and mammals (e.g., rat, mouse, guinea pig, rabbit, sheep, swine, bovine, horse, cat, dog, monkey, etc.).

The dose of the compound or its salt may vary depending on target disease, subject of administration, route for administration, etc. When the compound inhibiting the promoter activity for the DNA of the present invention is orally administered, the compound is administered to a patient (as 60 kg) with Alzheimer's disease, generally in a daily dose of approximately 0.1 to 100 mg, preferably approximately 1.0 mg to 50 mg, more preferably approximately 1.0 to 20 mg. When the compound is parenterally administered, a single dose of the compound may vary depending on subject of administration, target disease, etc. When the compound inhibiting the promoter activity for the DNA of the present invention is administered in the form of an injection to a patient (as 60 kg) with Alzheimer's disease, it is advantageous to administer the compound by intravenous injection in a daily dose of approximately 0.01 to 30 mg, preferably approximately 0.1 to 20 mg, more preferably approximately 0.1 to 10 mg. For other animal species, the corresponding dose as converted per 60 kg weight can be administered.

Thus, the non-human mammal deficient in expression of the DNA of the present invention is extremely useful in screening a compound or its salt that promotes or inhibits the activity of a promoter for the DNA of the present invention, and can contribute significantly to elucidation of causes for various diseases attributable to deficient expression of the DNA of the present invention or development of a prophylactic/therapeutic agent for the diseases.

Further, genes encoding various proteins are ligated downstream from DNA containing a promoter region for the protein of the present invention and injected into a fertilized egg of an animal to create a transgenic animal by which the protein of the present invention can be specifically synthesized and examined for its action in the living body. When a suitable reporter gene is ligated to the promoter region to establish a cell strain expressing the same, the cell strain can be used as a system of searching for a low-molecular compound having an action of specifically promoting or suppressing the ability of the cell strain to produce the protein of the present invention in vivo.

In the specification, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.
- DNA:: deoxyribonucleic acid
- cDNA :: complementary deoxyribonucleic acid
- A :: adenine
- T :: thymine
- G :: guanine
- C :: cytosine
- RNA:: ribonucleic acid
- mRNA :: messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP :: adenosine triphosphate
- EDTA :: ethylenediaminetetraacetic acid
- SDS :: sodium dodecyl sulfate
- Gly :: glycine
- Ala :: alanine
- Val :: valine
- Leu: leucine
- Ile: : isoleucine
- Ser :: serine
- Thr :: threonine
- Cys :: cysteine
- Met :: methionine
- Glu :: glutamic acid
- Asp :: aspartic acid
- Lys: lysine
- Arg :: arginine
- His :: histidine
- Phe :: phenylalanine
- Tyr :: tyrosine
- Trp :: tryptophan
- Pro :: proline
- Asn :: asparagine
- Gln :: glutamine
- pGlu :: pyroglutamic acid
- Sec :: selenocysteine

The substituents, protective groups and reagents, which are frequently used throughout the specification, are shown by the following abbreviations.
- Me :: methyl
- Et: : ethyl
- Bu :: butyl
- Ph :: phenyl
- TC :: thiazolidine-4(R)-carboxamide
- Tos :: p-toluenesulfonyl
- CHO:: formyl
- Bzl :: benzyl
- Cl₂Bzl:: 2,6-dichlorobenzyl
- Bom :: benzyloxymethyl
- Z :: benzyloxycarbonyl
- Cl-Z :: 2-chlorobenzyloxycarbonyl
- Br-Z :: 2-bromobenzyloxycarbonyl
- Boc :: t-butoxycarbonyl
- DNP :: dinitrophenol
- Trt :: trityl
- Bum :: t-butoxymethyl
- Fmoc: : N-9-fluorenylmethoxycarbonyl
- HOBt: : 1-hydroxybenztriazole
- HOOBt:: 3,4-dihydro-3-hydroxy-4-oxo-1,2,3-benzotriazine
- HONB :: 1-hydroxy-5-norbornene-2,3-dicarboxyimide
- DCC:: N,N'-dicyclohexylcarbodiimide

The sequence identification numbers in the sequence listing of the present specification indicate the following sequences, respectively.
SEQ ID NO: 1 shows the amino acid sequence of C1 protein.
SEQ ID NO: 2 shows the nucleotide sequence of DNA encoding C1 protein.
SEQ ID NO: 3 shows the nucleotide sequence of a primer for race used in Example 3.
SEQ ID NO: 4 shows the nucleotide sequence of a primer for nested race used in Example 3.
SEQ ID NO: 5 shows the nucleotide sequence of a primer used in Example 3.
SEQ ID NO: 6 shows the nucleotide sequence of a primer used in Example 3.
SEQ ID NO: 7 shows the nucleotide sequence of a primer used in Example 3.
SEQ ID NO: 8 shows the nucleotide sequence of a primer used in Example 3.
SEQ ID NO: 9 shows the nucleotide sequence of a primer used in Example 3.
SEQ ID NO: 10 shows the nucleotide sequence of a primer used in Example 3.

The transformant Escherichia coli DH5α/C1-pcDNA3.1/V5-His TOPO obtained in Example 4 described below has been deposited since May 22, 2003 under Accession No. FERM BP-8384 with International Patent Organisms Depository, National Institute of Advanced Industrial Science and Technology, located at Central 6, 1-1-1 Higashi, Tsukuba, Ibaraki Prefecture (zip code: 305-8566), Japan.

### EXAMPLES

Hereinafter, the present invention is described in more detail by reference to the Examples, which however are not intended to limit the scope of the present invention.

### Example 1

To clarify genes whose expression varied according to endoplasmic reticulum stress, the following experiment was conducted.

Rat primary nerve cells prepared from TP-17 CD rats (Nippon Charles River) were suspended in B27-containing neurobasal medium (NB medium, GIBCO), then put at a density of 250,000 cells/well in a type I collagen-coated 24-well plate (SUMILON), and cultured for 3 days. (i) Tunicamycin (Tnc) was added at a final concentration of 100 nM to the cultured cells, (ii) thapsigargin (Thap) was added at a final concentration of 2 nM to the cultured cells, or (iii) after the medium was exchanged twice with glucose-free B27-containing DMEM medium, 2-deoxyglucose (2DG) was added at a concentration of 3 mM to the cultured cells. After 4, 8, and 24 hours, total RNA was extracted from the respective cells by using RNeasy Mini Kit (Qiagen) according to its instructions. As the control, the cells to which each medium was added in place of the above chemicals (Tnc, Thap and 2DG) (cells to which no chemical was added) were used. These were used as the material to conduct gene expression analysis using an oligonucleotide microarray (Rat Genome U34A, Affymetrix).

The experimental method was in accordance with instructions of Affymetrix (Expression analysis technical manual). As a result of comparison of gene expression profiles of the cells stimulated with each chemical and the cells to which no chemical was added, the expression of GeneBank No. 170665 was promoted by endoplasmic reticulum stress stimulation [the above (i), (ii) and (iii)] (Table 1).

**[Table 1]**

| Cells | Gene expression level^{a} |
|---|---|
| After 4 hours of stimulation with tunicamycin | 3.00 |
| After 4 hours without stimulation | 0.66 |
| After 8 hours of stimulation with tunicamycin | 2.81 |
| After 8 hours without stimulation | 1.12 |
| After 24 hours of stimulation with tunicamycin | 3.63 |
| After 24 hours without stimulation | 0.97 |
| After 4 hours of stimulation with thapsigargin | 8.77 |
| After 4 hours without stimulation | 1.22 |
| After 8 hours of stimulation with thapsigargin | 9.65 |
| After 8 hours without stimulation | 1.18 |
| After 24 hours of stimulation with thapsigargin | 7.56 |
| After 24 hours without stimulation | 1.57 |
| After 4 hours after addition of 2DG | 16.71 |
| After 4 hours without stimulation | 1.26 |
| After 8 hours after addition of 2DG | 15.11 |
| After 8 hours without stimulation | 0.59 |
| After 24 hours after addition of 2DG | 14.55 |
| After 24 hours without stimulation | 1.15 |
| Gene expression level^{a} was standardized with the median (= 1) of the gene expression level of the gene showing presence detected by expression in the oligonucleotide microarray. | |

### Example 2

For the purpose of examining whether the expression of GeneBank No. 170665 whose expression was promoted by endoplasmic reticulum stress was influenced by β-amyloid stimulation, gene expression analysis was conducted using the cells stimulated with β-amyloid in the same manner as in Example 1.

Rat primary nerve cells prepared in the same manner as in Example 1 were suspended in N2-containing DMEM medium (GIBCO), then put at a density of 250,000 cells/well and cultured for 4 days. After culture, β-amyloid suspended at a final concentration of 25 nM in the above medium was added to the cells. After 4, 8, and 24 hours, total RNA was extracted from the cells and gene analysis was conducted in the same manner as in Example 1. As the control, the cells to which the above medium was added in place of β-amyloid were used. The expression of GeneBank No. 170665 was promoted in the cells stimulated with β-amyloid, as compared with the control (Table 2).

**[Table 2]**

| Cells | Gene expression level^{a} |
|---|---|
| After 4 hours of stimulation with β-amyloid | 3.44 |
| After 4 hours without stimulation | 1.84 |
| After 8 hours of stimulation with β-amyloid | 6.99 |
| After 8 hours without stimulation | 1.51 |
| After 24 hours of stimulation with β-amyloid | 4.40 |
| After 24 hours without stimulation | 1.76 |
| Gene expression level^{a} was standardized with the median (= 1) of the gene expression level of the gene showing presence detected by expression in the oligonucleotide microarray. | |

### Example 3

### (1) Identification of a human ortholog corresponding to GenBank No. AI170665

For the purpose of identification of a rat sequence comprising GenBank No. AI013865, race was carried out using rat cDNA as a template. The cDNA for race was synthesized in the following manner.

Rat primary nerve cells prepared in the same manner as in Example 1 were cultured for 3 days, and after tunicamycin was added to a final concentration of 500 nM, the cells were cultured for additional 24 hours, and total RNA was recovered using ISOGEN (Nippon Gene), and polyA RNA was purified using µMACS mRNA Isolation Kit (Miltenyi Biotec). Using this RNA as a template, cDNA for race was synthesized by using SMART RACE cDNA Amplification Kit (Clontech), and using this cDNA as a template, 5' race was conducted with a primer for race (SEQ ID NO:3) prepared on the basis of GenBank No. AI013865, and nested race was conducted with a race primer for nest (SEQ ID NO:4). The resulting race product was ligated with pCR4-TOPO (Invitrogen, Inc.) and used to transform Escherichia coli DH5α (TOYOBO). Using the resulting colonies, synthetic primers (SEQ ID NOS:5 and 6), and ExTaq (TAKARA) as an enzyme, PCR was carried out under the following conditions (1) to (3) to give a specific PCR product.
(1) reaction at 96°C for 1 minute,
(2) 30 cycles each consisting of reaction at 96°C for 15 seconds, at 60°C for 10 seconds and at 72°C for 3 minutes, and
(3) reaction at 72°C for 5 minutes.

A substrate in this PCR product was decomposed with ExoSAP-IT (Amersham Pharmacia), and this sample was used as a template to conduct sequencing reaction with BigDye Terminator Cycle Sequencing Ready Reaction (ABI), and the sequenced product was analyzed by 3100 Genetic analyzer (ABI). When the resulting sequence was examined for homology, it had high homology with MGC4504 (Genbank Accession No. BC019625). When it was searched for in the Celera database, it had high homology with a hypothetical transcriptional sequence hCT30212. When the two were compared, MGC4504 was found to contained in the C-terminal region of ORF present in hCT30212.

From the foregoing result, it was estimated that a human ortholog of GenBank No. AI013865 was hCT30212, and cloning of ORF in hCT30212 was attempted.

### (2) Cloning of ORF in hCT30212

Thapsigargin was added at a final concentration of 500 nM to human neuroblastoma SK-N-AS cells (purchased from ATCC) and the cells were cultured for 8 hours. After culture, total RNA was extracted with ISOGEN (Nippon Gene). Using the resulting total RNA as a template, reverse transcription reaction was conducted with RNA PCR kit (TAKARA). For amplification of hCT30212 ORF, PCR was conducted using synthetic primers (SEQ ID NOS:7 and 8) and Pfu turbo (Stratagene) as an enzyme under the following conditions (1) to (6) to give a specific PCR product.
(1) reaction at 95°C for 30 seconds,
(2) 3 cycles each consisting of reaction at 95°C for 10 seconds, at 68°C for 10 seconds and at 72°C for 2 minutes,
(3) 3 cycles each consisting of reaction at 95°C for 10 seconds, at 66°C for 10 seconds and at 72°C for 2 minutes,
(4) 3 cycles each consisting of reaction at 95°C for 10 seconds, at 64°C for 10 seconds and at 72°C for 2 minutes,
(5) 35 cycles each consisting of reaction at 95°C for 10 seconds, at 62°C for 10 seconds and at 72°C for 2 minutes, and
(6) reaction at 72°C for 5 minutes.

The resulting PCR product was cloned into pcDNA3.1/V5-His TOPO (Invitrogen) and used to transform Escherichia coli DH5α. Using the resulting colonies, synthetic primers (SEQ ID NOS:9 and 10), and ExTaq (TAKARA) as an enzyme, PCR was carried out to give a specific PCR product. A substrate in this PCR product was decomposed with ExoSAP-IT (Amersham Pharmacia), and this sample was used as a template to conduct sequencing reaction with BigDye Terminator Cycle Sequencing Ready Reaction (ABI), and the sequenced product was analyzed by 3100 Genetic analyzer (ABI). The resulting nucleotide sequence (SEQ ID NO:2) encoded a 264-amino-acid sequence (SEQ ID NO:1) corresponding to hypothetical ORF in hCT30212, and a protein comprising the amino acid sequence was designated C1 protein.

C1 shows 84% homology at the amino acid and base level with hypothetical protein MGC4504 (Genbank No. BC019625) reported in humans.

### Example 4

A plasmid comprising the nucleotide sequence represented by SEQ ID NO:2 was designated C1-pcDNA3.1/V5-His TOPO, and a transformant having the plasmid was designated Escherichia coli DH5α/C1-pcDNA3.1/V5-His TOPO. This transformant was cultured in LB medium, and the vector was recovered by using QUAGEN plasmid midi kit (Qiagen). This C1 gene expression vector was transformed into SK-N-AS cells by using Nucleofector (AMAXA). As the control, pcDNA3.1 (Invitrogen) was transformed into SK-N-AS cells.

The respective transformed cells were put at a density of 7500 cells/well in a type I collagen-coated 96-well plate (IWAKI) and then cultured overnight, and after tunicamycin was added at various concentrations, the cells were cultured for 1 or 2 days to induce cell-death. After culture, DNA cleavage accompanying cell-death was detected by using CELL DEATH DETECTION ELISA PLUS kit (Roche). The experimental method was in accordance with instructions attached to the kit.

The results are shown in Figs. 1 and 2. DNA cleavage was promoted in the cells transformed with the C1 gene, as compared with the SK-N-AS cells transformed with pcDNA3.1 (control cells).

From the result, it is evident that C1 had an cell-death promoting action.

### Example 5

The C1 gene expression vector described in Example 4 was transformed into IMR-32 cells by using Nucleofector (AMAXA). As the control, a GFP gene expression vector (Wako) was transformed into other IMR-32 cells. The respective transformed cells were put at a density of 1,000,000 cells/well in a type I collagen-coated 24-well plate (IWAKI) and cultured for 16 hours. After the medium was exchanged with a new one, the cells were cultured for additional 24 hours, and the concentrations of Aβ40 and Aβ42 in the culture supernatant were measured respectively by ELISA (Biochemistry 34, 10272-10278, 1995).

The results are shown in Figs. 3 and 4.

Aβ40 concentration (Fig. 3) and Aβ42 concentration (Fig. 4) were reduced in the culture supernatant of the cells transformed with C1, as compared with the IMR-32 cells transformed with GFP (control cells).

From the result, it is evident that C1 had an inhibitory action on secretion of Aβ40 and Aβ42.

### INDUSTRIAL APPLICABILITY

The protein of the present invention, the polynucleotide of the present invention, the antisense polynucleotide of the present invention, or the antibody of the present invention, which has an cell-death promoting activity, a β-amyloid production-inhibiting activity, etc., is useful as a diagnostic marker for neurodegenerative diseases (for example, Alzheimer's disease [e.g., familial Alzheimer's disease, juvenile Alzheimer's disease, sporadic Alzheimer's disease etc.], Parkinson's disease, Down's syndrome, amyotrophic lateral sclerosis, prion disease, Creutzfeldt-Jakob disease, Huntington's disease, diabetic neuropathy, multiple sclerosis etc.). A compound promoting or inhibiting the activity of the protein, a compound promoting or inhibiting the expression of the gene for the protein, etc. can be used as a safe pharmaceutical such as a prophylactic/therapeutic agent for neurodegenerative diseases (for example, Alzheimer's disease [e.g., familial Alzheimer's disease, juvenile Alzheimer's disease, sporadic Alzheimer's disease, mild cognitive impairment etc.], cerebral amyloid angiopathy, Parkinson's disease, Down's syndrome, amyotrophic lateral sclerosis, prion disease, Creutzfeldt-Jakob disease, Huntington's disease, diabetic neuropathy, multiple sclerosis etc.). A compound promoting the activity of the protein, a compound promoting the expression of the gene for the protein, etc. can be used for example as a β-amyloid production inhibitor etc. A compound inhibiting the activity of the protein, a compound inhibiting the expression of the gene for the protein, etc. can be used for example as an cell-death inhibitor etc. The protein, polynucleotide and antibody according to the present invention are useful for screening the pharmaceuticals described above.

## Claims

1. A protein comprising the same or substantially the same amino acid sequence as an amino acid sequence represented by SEQ ID NO: 1, or a salt thereof.

2. A protein consisting of an amino acid sequence represented by SEQ ID NO: 1, or a salt thereof.

3. A partial peptide of the protein according to claim 1, or a salt thereof.

4. A polynucleotide comprising a polynucleotide encoding the protein according to claim 1 or the partial peptide according to claim 3.

5. The polynucleotide according to claim 4, which is DNA.

6. A polynucleotide consisting of a nucleotide sequence represented by SEQ ID NO: 2.

7. A recombinant vector comprising the polynucleotide according to claim 5.

8. A transformant transformed with the recombinant vector according to claim 7.

9. A method of manufacturing the protein or its salt according to claim 1 or the partial peptide or its salt according to claim 3, which comprises culturing the transformant according to claim 8, forming and accumulating the protein according to claim 1 or the partial peptide according to claim 3, and recovering it.

10. A pharmaceutical comprising the protein or its salt according to claim 1 or the partial peptide or its salt according to claim 3.

11. A pharmaceutical comprising the polynucleotide according to claim 5.

12. A diagnostic agent comprising the polynucleotide according to claim 5.

13. An antibody to the protein according to claim 1, the partial peptide according to claim 3, or a salt of the protein or partial peptide.

14. An antisense polynucleotide comprising a nucleotide sequence, or a part thereof, complementary or substantially complementary to the nucleotide sequence of a polynucleotide encoding a protein or its partial peptide thereof comprising the same or substantially the same amino acid sequence as an amino acid sequence represented by SEQ ID NO: 1.

15. A pharmaceutical comprising the antisense polynucleotide according to claim 14.

16. A method of screening a compound or its salt that promotes or inhibits the activity of the protein or its salt according to claim 1 or the partial peptide or its salt according to claim 3, which comprises using the protein or its salt according to claim 1 or the partial peptide or its salt according to claim 3.

17. A kit for screening a compound or its salt that promotes or inhibits the activity of the protein or its salt according to claim 1 or the partial peptide or its salt according to claim 3, which comprises the protein or its salt according to claim 1 or the partial peptide or its salt according to claim 3.

18. A method of screening a compound or its salt that promotes or inhibits the expression of a gene for the protein according to claim 1, which comprises using the polynucleotide according to claim 4.

19. A kit for screening a compound or its salt that promotes or inhibits the expression of a gene for the protein according to claim 1, which comprises the polynucleotide according to claim 4.

20. A diagnostic agent comprising the antibody according to claim 13.

21. A pharmaceutical comprising the antibody according to claim 13.

22. A method of quantifying the protein according to claim 1, which comprises using the antibody according to claim 13.

23. A method of diagnosing diseases associated with the functions of the protein according to claim 1, which comprises using the quantification method according to claim 22.

24. A method of screening a compound or its salt that promotes or inhibits the expression of the protein according to claim 1, which comprises using the antibody according to claim 13.

25. A kit for screening a compound or its salt that promotes or inhibits the expression of the protein according to claim 1, which comprises the antibody according to claim 13.

26. The pharmaceutical according to claim 10, 11, 15 or 21, which is a prophylactic/therapeutic agent for a neurodegenerative disease.

27. The diagnostic agent according to claim 12 or 20, which is a diagnostic agent for a neurodegenerative disease.

28. A prophylactic/therapeutic method for a neurodegenerative disease, which comprises administering to a mammal an effective amount of a compound or its salt that promotes or inhibits the activity of the protein according to claim 1 or its partial peptide or its salt, a compound or its salt that promotes or inhibits the expression of a gene for said protein or its partial peptide or its salt, or a compound or its salt that promotes or inhibits the expression of said protein or its partial peptide or its salt.

29. A method of inhibiting production of β-amyloid, which comprises administering to a mammal an effective amount of a compound or its salt that promotes the activity of the protein according to claim 1 or its partial peptide or its salt, a compound or its salt that promotes the expression of a gene for said protein or its partial peptide or its salt, or a compound or its salt that promotes the expression of said protein or its partial peptide or its salt.

30. A method of inhibiting cell-death, which comprises administering to a mammal an effective amount of a compound or its salt that inhibits the activity of the protein according to claim 1 or its partial peptide or its salt, a compound or its salt that inhibits the expression of a gene for said protein or its partial peptide or its salt, or a compound or its salt that inhibits the expression of said protein or its partial peptide or its salt.

31. A prophylactic/therapeutic method for a neurodegenerative disease, wherein the activity of the protein according to claim 1 or its partial peptide or its salt is promoted or inhibited, the expression of a gene for said protein or its partial peptide or its salt is promoted or inhibited, or the expression of said protein or its partial peptide or its salt is promoted or inhibited.

32. A method of inhibiting production of β-amyloid, wherein the activity of the protein according to claim 1 or its partial peptide or its salt is promoted, the expression of a gene for said protein or its partial peptide or its salt is promoted, or the expression of said protein or its partial peptide or its salt is promoted.

33. A method of inhibiting cell-death, wherein the activity of the protein according to claim 1 or its partial peptide or its salt is inhibited, the expression of a gene for said protein or its partial peptide or its salt is inhibited, or the expression of said protein or its partial peptide or its salt is inhibited.

34. Use of a compound or its salt that promotes or inhibits the activity of the protein according to claim 1 or its partial peptide or its salt, a compound or its salt that promotes or inhibits the expression of a gene for said protein or its partial peptide or its salt, or a compound or its salt that promotes or inhibits the expression of said protein or its partial peptide or its salt, for the manufacture of a prophylactic/therapeutic agent for a neurodegenerative disease.

35. Use of a compound or its salt that promotes the activity of the protein according to claim 1 or its partial peptide or its salt, a compound or its salt that promotes the expression of a gene for said protein or its partial peptide or its salt, or a compound or its salt that promotes the expression of said protein or its partial peptide or its salt, for the manufacture of a β-amyloid production inhibitor.

36. Use of a compound or its salt that inhibits the activity of the protein according to claim 1 or its partial peptide or its salt, a compound or its salt that inhibits the expression of a gene for said protein or its partial peptide or its salt, or a compound or its salt that inhibits the expression of said protein or its partial peptide or its salt, for the manufacture of an cell-death inhibitor.


SEQUENCE LISTING

<110> Takeda Pharmaceutical Company Limited

<120> Novel protein and its DNA

<130> G05-0048

<140> PCT/JP2004/003033
<141> 2004-03-09

<150> JP2003-64197
<151> 2003-03-10

<150> JP2003-149679
<151> 2003-05-27

<160> 10

<210> 1
<211> 264
<212> PRT
<213> Homo sapiens

<400> 1
Met Gly Gly Ala Gln Leu Glu Leu Pro Ser Gly Ala Arg Pro Gly Val
                5                   10                  15
Cys Val Arg Arg Ser Phe Arg Ala His Ala Gly Asp Gln Pro Arg Arg
            20                  25                  30

Pro Pro Gly Pro Ile Pro Val Pro Gly Thr Met Lys Gln Glu Ser Ala
        35                  40                  45
Ala Pro Asn Thr Pro Pro Thr Ser Gln Ser Pro Thr Pro Ser Ala Gln
    50                  55                  60
Phe Pro Arg Asn Asp Gly Asp Pro Gln Ala Leu Trp Ile Phe Gly Tyr
65                  70                  75                  80
Gly Ser Leu Val Trp Arg Pro Asp Phe Ala Tyr Ser Asp Ser Arg Val
                85                  90                  95
Gly Phe Val Arg Gly Tyr Ser Arg Arg Phe Trp Gln Gly Asp Thr Phe
            100                 105                 110
His Arg Gly Ser Asp Lys Met Pro Gly Arg Val Val Thr Leu Leu Glu
        115                 120                 125
Asp His Glu Gly Cys Thr Trp Gly Val Ala Tyr Gln Val Gln Gly Glu
    130                 135                 140
Gln Val Ser Lys Ala Leu Lys Tyr Leu Asn Val Arg Glu Ala Val Leu
145                 150                 155                 160
Gly Gly Tyr Asp Thr Lys Glu Val Thr Phe Tyr Pro Gln Asp Ala Pro
                165                 170                 175
Asp Gln Pro Leu Lys Ala Leu Ala Tyr Val Ala Thr Pro Gln Asn Pro
            180                 185                 190
Gly Tyr Leu Gly Pro Ala Pro Glu Glu Ala Ile Ala Thr Gln Ile Leu
        195                 200                 205
Ala Cys Arg Gly Phe Ser Gly His Asn Leu Glu Tyr Leu Leu Arg Leu
    210                 215                 220
Ala Asp Phe Met Gln Leu Cys Gly Pro Gln Ala Gln Asp Glu His Leu
225                 230                 235                 240
Ala Ala Ile Val Asp Ala Val Gly Thr Met Leu Pro Cys Phe Cys Pro
                245                 250                 255
Thr Glu Gln Ala Leu Ala Leu Val

            260

<210> 2

<211> 792

<212> DNA

<213> Homo sapiens

<400> 2

atggggggcg ctcagctgga gctaccgagc ggtgccaggc caggtgtgtg cgtccgtcgg 60

tctttccgtg cccacgccgg agaccagccc cggaggccgc ctgggcctat ccctgtgcca 120

ggcaccatga agcaggagtc tgcagccccg aacaccccgc ccacctcgca gtcccctacg 180

ccgtccgctc agttcccccg aaacgacggc gaccctcaag cgctgtggat tttcgggtac 240

ggctccctgg tgtggaggcc cgacttcgcc tacagcgaca gccgtgtggg cttcgtgcgc 300

ggctacagcc gccgtttctg gcagggagac accttccatc ggggcagcga caagatgcct 360

ggccgtgtgg tgacgctcct tgaagatcat gagggctgca cttggggcgt ggcataccaa 420

gtgcaagggg agcaggtaag caaggccctg aagtacctga atgtgcgaga ggcagtgctt 480

ggtggctacg ataccaagga ggtcaccttc tatccccaag atgctcctga ccaaccactg 540

aaggcattgg cctatgtggc caccccacag aaccctggtt acctgggccc tgcgcctgaa 600

gaggccattg ccacgcagat cctggcctgc cggggcttct ccggccacaa ccttgaatac 660

ttgctgcgtc tggcagactt catgcagctc tgtgggcctc aggcgcagga cgagcacctg 720

gcagccatcg tggacgctgt gggcaccatg ttgccctgct tctgccccac cgagcaggct 780

ctggcgctgg tg 792

<210> 3

<211> 18

<212> DNA

<213> Artificial Sequence

<220>

<223> Primer

<400> 3
ggcaggggta taagacac 18

<210> 4
<211> 19
<212> DNA
<213> Artificial Sequence

<220>
<223> Primer

<400> 4
acccgccatg ccttctcag 19

<210> 5
<211> 24
<212> DNA
<213> Artificial Sequence

<220>
<223> Primer

<400> 5
cgccagggtt ttcccagtca cgac 24

<210> 6
<211> 27

<212> DNA
<213> Artificial Sequence

<220>
<223> Primer

<400> 6
agcggataac aatttcacac aggaaac 27

<210> 7
<211> 30
<212> DNA
<213> Artificial Sequence

<220>
<223> Primer

<400> 7
gccatggggg gcgctcagct ggagctaccg 30

<210> 8
<211> 26
<212> DNA
<213> Artificial Sequence

<220>
<223> Primer

<400> 8

tcacaccagc gccagagcct gctcgg 26

<210> 9

<211> 20

<212> DNA

<213> Artificial Sequence

<220>

<223> Primer

<400> 9

taatacgact cactataggg 20

<210> 10

<211> 18

<212> DNA

<213> Artificial Sequence

<220>

<223> Primer

<400> 10

tagaaggcac agtcgagg 18